# EUROPÄISCHE PATENTSCHRIFT

(11) **EP 4 150 745 B1**
(45) Veröffentlichungstag und Bekanntmachung des Hinweises auf die Patenterteilung: **23.09.2026**
(21) Anmeldenummer: 21725517.3
(22) Anmeldetag: 12.05.2021
(51) Int. Cl.: H02K 15/35, B23K 31/12, B23K 26/22, B23K 101/38, G01N 25/72, G01N 33/207, G01N 21/17

(54) **VERFAHREN ZUR ÜBERWACHUNG EINER ANBINDUNGSFLÄCHE BEIM LASERSCHWEISSEN VON KUPFERHALTIGEN, GEBOGENEN STABLEITERN**
METHOD FOR MONITORING OF CONNECTION AREAS IN LASER WELDING OF CONDUCTOR BARS CONTAINING COPPER
MÉTHODE DE CONTRÔLE DE LA ZONE DE RACCORDEMENT DE SOUDAGE PAR LASER DE BARRES CONDUCTRICES CONTENANT DU CUIVRE

(30) Priorität: 15.05.2020 DE 102020113179
(43) Veröffentlichungstag der Anmeldung: 22.03.2023
(73) Patentinhaber: TRUMPF Laser- und Systemtechnik SE, 71254 Ditzingen (DE)
(72) Erfinder: BOCKSROCKER, Oliver, 74343 Sachsenheim (DE); SPEKER, Nicolai, 74385 Pleidelsheim (DE)
(74) Vertreter: Trumpf Patentabteilung
(86) Internationale Anmeldenummer: PCT/EP2021/062708
(87) Internationale Veröffentlichungsnummer: WO 2021/228989

(56) Entgegenhaltungen:
- DE-A1- 102016 211 782
- JP-A- 2002 346 776
- US-A1- 2006 249 487
- US-A1- 2013 075 371
- US-A1- 2019 210 158

## Beschreibung

Die Erfindung betrifft ein Verfahren zur Überwachung einer Anbindungsfläche beim Laserschweißen von kupferhaltigen, gebogenen Stableitern, insbesondere von Hairpins für einen Elektromotor,
wobei zwei Stableiter teilweise überlappend angeordnet werden und mittels eines Bearbeitungs-Laserstrahls miteinander verschweißt werden,
wobei sich eine Schweißperle ausbildet, durch die die Stableiter miteinander verbunden werden.

Allgemeine Verfahren zur Beobachtung und/oder zur Beurteilung von Schweißverbindungen sind beispielsweise aus der JP 2002 346776 A, der US 2006/249487 A1, der DE 10 2016 211782 A1, der US 2013/075371 A1, oder der US 2019/210158 A1 bekannt.

Kupferhaltige, gebogene Stableiter, insbesondere so genannte Hairpins, werden in elektrodynamischen Maschinen, wie Elektromotoren oder elektrischen Generatoren, verbaut. Die Stableiter werden, entsprechend einer vorgesehenen elektrischen Verschaltung, angeordnet und miteinander verschweißt, um damit einen Elektromagneten aufzubauen. Eine elektrodynamische Maschine verfügt dabei typischerweise über mehrere Dutzend, oft Hunderte von gebogenen Stableitern, die paarweise miteinander verschweißt werden müssen.

Dabei ist es wichtig, mittels der Verschweißung eine ausreichende Querschnittsfläche zur Verfügung zu stellen, durch die der elektrische Strom von einem Stableiter in den anderen Stableiter fließen kann ("Anbindungsfläche"). Ist die Anbindungsfläche zu klein, droht im Betrieb eine erhebliche ohmsche Erwärmung, eine Einbuße im Wirkungsgrad oder gar eine Unbrauchbarkeit der elektrodynamischen Maschine.

Das Verschweißen der Stableiter erfolgt oftmals mittels eines Laserstrahls ("Laserschweißen"). Ein Laserstrahl wird dafür typischerweise auf die endseitigen Stirnflächen von zwei überlappenden, meist aneinander anliegenden Stableitern gerichtet, wodurch Wärme in die Stableiter eingebracht wird, diese aufschmelzen und nach der Erstarrung über die wieder erstarrte Schweißperle miteinander verbunden sind. In der Regel wird der Laserstrahl mit einer vorgegebenen Leistung für eine vorgegebene Zeit auf die Stableiter gerichtet, wodurch in der Regel eine ausreichend große Anbindungsfläche erreicht wird.

Aufgrund von Verschmutzungen oder Rauigkeit an der Oberfläche der Stableiter kann jedoch die Reflektivität der Stableiter für den Laserstrahl schwanken, und dadurch auch der tatsächliche Energieeintrag. Ebenso kann durch Fehlpositionierungen der Stableiter, etwa Spalten oder Versatz, oder ungenauer Positionierung des Laserstrahls der tatsächliche Energieeintrag variieren. Bei zu geringem Energieeintrag wird zu wenig Material aufgeschmolzen, so dass eine zu kleine Schweißperle entsteht, die eine zu geringe Anbindungsfläche zur Verfügung stellt. Auch im Falle von starker Spritzerbildung beim Laserschweißen kann eine zu kleine Schweißperle mit zu geringer Anbindungsfläche entstehen.

Die Größe der erreichten Anbindungsfläche kann mittels Röntgenanalyse bestimmt werden. Dafür muss der Stableiter nach dem Verschweißen in eine geeignete Röntgenapparatur zur Anfertigung des Röntgenbildes verbracht werden, was apparativ aufwändig ist. Ebenso ist es bekannt, von der Schweißperle einen metallographischen Querschliff anzufertigen, was jedoch recht aufwändig ist und die Verschweißung zerstört.

### Aufgabe der Erfindung

Es ist Aufgabe der Erfindung, ein Verfahren zur Überwachung der Anbindungsfläche beim Laserschweißen von kupferhaltigen, gebogenen Stableitern zur Verfügung zu stellen, welches einfach, schnell und zerstörungsfrei durchzuführen ist.

### Beschreibung der Erfindung

Diese Aufgabe wird erfindungsgemäß gelöst durch ein Verfahren nach Anspruch 1, nämlich ein Verfahren der eingangs genannten Art, bei dem nach dem Ende der Einwirkung des Bearbeitungs-Laserstrahls während des Abkühlens der Schweißperle wenigstens eine mit der Temperatur der Schweißperle veränderliche Messvariable zumindest an einem Teil der Schweißperle als Funktion der Zeit gemessen wird.

Erfindungsgemäß wird aus der wenigstens einen gemessenen Messvariablen ein von der Wärmekapazität der Schweißperle abhängiger Parameter bestimmt, und aus dem Parameter die Anbindungsfläche qualitativ oder quantitativ bestimmt.

Im Rahmen des erfindungsgemäßen Verfahrens wird die Größe bzw. die Masse der Schweißperle auf indirekte Weise über die Ableitung der durch das Laserschweißen eingebrachten Wärme aus der Schweißperle nach dem Ende des Laserschweißvorgangs bestimmt. Im Allgemeinen kann dann von der Größe der Schweißperle auf die Größe der Anbindungsfläche rückgeschlossen werden, und damit die Anbindungsfläche überwacht werden.

Man beachte, dass eine direkte Bestimmung der Größe der Schweißperle, etwa durch Analyse eines optischen Bildes der Schweißperle, aufgrund von Unregelmäßigkeiten an den Stableitern, insbesondere Schrägflächen an den Stableiterenden, nach den Erfahrungen der Erfinder in der Regel nicht mit ausreichender Zuverlässigkeit möglich ist.

Die Schweißperle hat am Ende der Einwirkung des Bearbeitungs-Laserstrahls näherungsweise die Verdampfungstemperatur des Stableitermaterials; entsprechend ist näherungsweise eine der Masse der Schweißperle proportionale Wärmemenge in der Schweißperle gespeichert.

Diese gespeicherte Wärmemenge wird über die in der gegebenen Schweißsituation vorhandenen Wärmeleitpfade abgeleitet, wobei die Wärmeleitpfade durch die Dimensionen der Stableiter und das Stableitermaterial vorgegeben sind, und (bei normalem Schweißverlauf, d.h. Kontakt der Schweißperle zu den Stableitern über deren vollen Querschnitt) nicht von der Größe der Schweißperle abhängen. In guter Näherung erfolgt die Ableitung der in der Schweißperle gespeicherten Wärmemenge eindimensional durch die beiden Stableiter. Der Wärmestrom durch die Stableiter ist dabei begrenzt.

Je größer die ursprünglich gespeicherte Wärmemenge der Schweißperle ist, desto langsamer erfolgt die Abkühlung der Schweißperle. Mittels der Messvariablen wird das Abkühlverhalten beobachtet. Über das Abkühlverhalten kann damit auf die ursprüngliche gespeicherte Wärmemenge rückgeschlossen werden, und damit auf die (absolute) Wärmekapazität.

Die Messvariable, die eine durch die Temperatur direkt oder indirekt (etwa über den Phasenzustand) beeinflusste Eigenschaft zumindest des Teils der Schweißperle beschreibt, wird als Funktion der Zeit gemessen; dadurch wird das Abkühlverhalten der Schweißperle beobachtet, und der von der (absoluten) Wärmekapazität der Schweißperle abhänge Parameter kann bestimmt werden. Der von der (absoluten) Wärmekapazität abhängige Parameter indiziert die Masse der Schweißperle, da die (absolute) Wärmekapazität der Schweißperle proportional zur Masse der Schweißperle ist.

Die Masse der Schweißperle bestimmt wiederum deren geometrische Größe, und damit die Anbindungsfläche, die die Schweißperle bzw. die Verschweißung zwischen den Stableitern insbesondere zur Durchleitung von elektrischem Strom zur Verfügung stellt. Über eine Kalibrierung für die gegebene Schweißsituation (insbesondere für die Dimensionen der Stableiter und das Stableitermaterial) kann dann erfindungsgemäß aus dem Parameter auf die Anbindungsfläche geschlossen werden.

Zur Kalibrierung der Überwachung der Anbindungsfläche kann für einige Verschweißungen jeweils die Messvariable vermessen und daraus der Parameter bestimmt werden, und weiterhin jeweils die tatsächliche Anbindungsfläche konventionell (etwa durch eine Röntgenuntersuchung oder einen metallographischen Querschliff) bestimmt werden ("Kalibrierexperimente"). Aus den Kalibrierexperimenten ergibt sich dann (für die gegebene Schweißsituation) der Zusammenhang zwischen dem Parameter und der tatsächlichen Anbindungsfläche. Dieser Zusammenhang kann dann bei späteren Verschweißungen (bei gleicher Schweißsituation) dazu genutzt werden, die Anbindungsfläche qualitativ oder quantitativ aus dem Parameter zu bestimmen, ohne dass dann noch eine konventionelle Bestimmung der tatsächlichen Anbindungsfläche erfolgt.

Eine typische qualitative Bestimmung der Anbindungsfläche beschränkt sich auf eine Aussage, ob die Anbindungsfläche ausreichend groß ist (Verschweißung "iO", in Ordnung) oder nicht ausreichend groß ist (Verschweißung "niO", nicht in Ordnung). Eine typische quantitative Aussage ist eine unmittelbare Flächenangabe zur Anbindungsfläche (zum Beispiel "5,5 mm²).

### Bevorzugte Varianten der Erfindung

### Allgemeine Varianten

Bei einer bevorzugten Variante des erfindungsgemäßen Verfahrens ist vorgesehen,
dass die zwei Stableiter mit Endbereichen parallel zueinander und nebeneinander liegend angeordnet werden,
insbesondere wobei die Endbereiche der Stableiter flächig aneinander gepresst werden,
dass stirnseitige Endflächen der Stableiter näherungsweise auf gleicher Höhe bezüglich der Richtung einer Längserstreckung der Endbereiche der Stableiter liegen,
und dass der Bearbeitungs-Laserstrahl so auf die zwei Stableiter gerichtet wird, dass sich die Schweißperle an den stirnseitigen Endflächen der Stableiter ausbildet,
insbesondere wobei die Endbereiche der Stableiter näherungsweise vertikal nach oben gerichtet sind und der Bearbeitungs-Laserstrahl näherungsweise senkrecht auf die stirnseitigen Endflächen fällt. Bei Ausbildung der Schweißperle an den stirnseitigen Endflächen der Stableiter ist der Wärmeabfluss aus der Schweißperle gut definiert, und das erfindungsgemäße Verfahren ist besonders präzise. Typischerweise wird der Bearbeitungs-Laserstrahl direkt auf die stirnseitigen Endflächen gerichtet, und bewegt sich dabei zwischen den Stableitern hin und her, etwa auf einer Kreisbahn. Bei einer Ausrichtung der Endbereiche (Beine) der Stableiter vertikal nach oben liegt die darauf erzeugte (zunächst noch flüssige) Schweißperle besonders stabil.

Besonders bevorzugt ist eine Variante, bei der zur Messung der Messvariable zumindest ein Teil der Schweißperle im sichtbaren Spektralbereich und/oder im infraroten Spektralbereich beobachtet wird. Eine Beobachtung im sichtbaren oder infraroten Spektralbereich ist bei geringen Kosten leicht möglich, etwa mit einer Kamera oder einer Photodiode. Bei vielen Schweißapparaturen sind optische Beobachtungssysteme bereits vorhanden, etwa zur relativen Ausrichtung von Laserstrahl und Werkstück(en), und können für das erfindungsgemäße Verfahren mit genutzt werden. Man beachte, dass die Beobachtung bevorzugt mit einer Frequenz von wenigstens 100 Hz erfolgt.

Bei einer bevorzugten Weiterentwicklung dieser Variante wird die wenigstens eine Messvariable an einem Teil der Schweißperle gemessen, welcher Teilflächen der Stableiter, auf die der Bearbeitungs-Laserstrahl eingewirkt hat, gegenüber liegt,
insbesondere wobei die wenigstens eine Messvariable an einem Teil der Schweißperle gemessen wird, der zentral und oben an der Schweißperle liegt. Durch die Messung der Messvariable an diesem Teil kann die Wärmeableitung besonders zuverlässig beobachtet werden; insbesondere sind Randeffekte durch Unregelmäßigkeiten der Stableiter minimiert. Zentral oben an der Schweißperle wird die Erstarrung der Schweißperle typischerweise abgeschlossen, so dass die Erstarrungszeit hier besonders leicht zu bestimmen ist.

### Varianten betreffend die Messvariable

Bei einer vorteilhaften Variante umfasst die wenigstens eine Messvariable eine Intensität einer Glühemission zumindest des Teils der Schweißperle. Die Intensität der Glühemission ist vergleichsweise einfach zu messen und auszuwerten.

Eine vorteilhafte Weiterentwicklung hierzu sieht vor, dass die Intensität der Glühemission lediglich in einem begrenzten Spektralbereich gemessen wird, insbesondere wobei der begrenzte Spektralbereich nicht größer als 800 nm bis 1100 nm ist,
insbesondere wobei zur Einschränkung des begrenzten Spektralbereichs ein Bandpassfilter und/oder einen Breitbandfilter vor einer Sensoreinrichtung zur Messung der Intensität der Glühemission angeordnet wird,
und insbesondere wobei ein Wellenlängenbereich des Bearbeitungs-Laserstrahls außerhalb des begrenzten Spektralbereichs liegt. Der Spektralbereich von 800 nm bis 1100 nm ist für kupferhaltige Stableiter für die Bestimmung der von der Temperatur abhängigen Messvariablen besonders aufschlussreich. Durch die Blockade der Wellenlänge des Bearbeitungs-Laserstrahls kann eine Überlastung der eingesetzten Sensoreinrichtung vermieden werden. Bei einer Wellenlänge des Bearbeitungs-Laserstrahls von ca. 1030 nm kann beispielsweise das gemessene Spektrum mit einem Bandpassfilter auf 800-1000 nm eingeschränkt werden, oder mit einem Breitbandfilter der Wellenlängenbereich um 1030 nm gezielt geblockt werden. Insbesondere kann auch ein gemessener Spektralbereich <1000 nm ausgewählt werden.

Bevorzugt ist eine Variante, die vorsieht,
dass während des Abkühlens der Schweißperle die Schweißperle mit einem Beobachtungs-Lichtstrahl, insbesondere Beobachtungs-Laserstrahl, beleuchtet wird, und dass die wenigstens eine Messvariable eine Intensität des an der Oberfläche der Schweißperle reflektierten Beobachtungs-Lichtstrahls umfasst. Bei der Abkühlung der Schweißperle, und insbesondere bei einem Phasenübergang (Erstarrung), ändert sich deren Reflektivität für den Beobachtungs-Lichtstrahl, was durch diese Variante genutzt werden kann. Typischerweise ist der Beobachtungs-Lichtstrahl schmalbandig (zB mit einer vollen spektralen Breite von 40 nm oder weniger, meist 20 nm oder weniger), und stammt bevorzugt aus einem Beobachtungs-Laser, insbesondere Dioden-Laser, oder einer LED oder einem LED-Ring. Durch Einsatz des Beobachtungs-Lichtstrahls kann eine besonders genaue Überwachung der Anbindungsfläche erfolgen. Man beachte, dass in der Praxis der reflektierte Beobachtungs-Lichtstrahl von der Glühemission der Schweißperle überlagert wird, und beide Effekte (bzw. zugehörige Intensitäten) grundsätzlich gemeinsam gemessen werden, etwa als mittlerer Grauwert in einem Teilbereich eines Bildes einer Kamera oder als Grauwert einer Photodiode.

Bevorzugt ist eine Weiterentwicklung dieser Variante, die vorsieht,
dass die Intensität des reflektierten Beobachtungs-Lichtstrahls lediglich in einem begrenzten Spektralbereich um eine mittlere Wellenlänge des Beobachtungs-Lichtstrahls gemessen wird,
insbesondere wobei der begrenzte Spektralbereich nicht größer als +/-20 nm oder +/- 10 nm um die mittlere Wellenlänge des Beobachtungs-Lichtstrahls ist, und insbesondere wobei zur Einschränkung des begrenzten Spektralbereichs vor einer Sensoreinrichtung, mit der die Intensität des reflektierten Beobachtungs-Lichtstrahls gemessen wird, ein Bandpassfilter angeordnet wird. Durch die Beschränkung auf die begrenzten Spektralbereich um die mittlere Wellenlänge des Beobachtungs-Lichtstrahls wird erreicht, dass (zusätzlich vorhandene) Glühemission die Messung der Messvariablen weniger stark überlagert bzw. verfälscht.

Ebenso bevorzugt ist eine Weiterentwicklung, bei der vor einer Sensoreinrichtung, mit der die Intensität des reflektierten Beobachtungs-Lichtstrahls gemessen wird, ein Polarisationsfilter angeordnet wird,
insbesondere wobei der Polarisationsfilter als ein Linienpolarisator gewählt ist. Dadurch können die Kontraste bei der Beobachtung der Schweißperle über den reflektierten Beobachtungs-Lichtstrahl verbessert werden.

Eine Weiterentwicklung der Varianten mit Beobachtung der Intensität der Glühemission und/oder des reflektierten Beobachtungs-Lichtstrahls sieht vor, dass die Schweißperle mit einer Kamera beobachtet wird, und die Intensität der Glühemission und/oder des reflektierten Beobachtungs-Lichtstrahls an einem Teil der Schweißperle mit der Kamera bestimmt wird, indem ein mittlerer Grauwert der Kamera in einem Teilbereich des von der Kamera aufgenommenen Bildes bestimmt wird. Dieses Vorgehen ist vergleichsweise einfach und in der Praxis bewährt; geeignete Kameras stehen bei einem Schweißaufbau oftmals ohnehin zur Verfügung, etwa zur Positionierung des Laserstrahls auf dem Werkstück.

Bei einer anderen Weiterentwicklung der Varianten mit Beobachtung der Intensität der Glühemission und/oder des reflektierten Beobachtungs-Lichtstrahls ist vorgesehen, dass die Schweißperle mit einer Photodiode beobachtet wird, und die Intensität der Glühemission und/oder des reflektierten Beobachtungs-Lichtstrahls zumindest an einem Teil der Schweißperle als der Grauwert der Photodiode bestimmt wird. Die Bestimmung des Grauwerts mit einer Photodiode ist besonders kostengünstig.

Bevorzugt ist weiterhin eine Variante, bei der die Messvariable eine Temperatur zumindest an einem Teil der Schweißperle umfasst. Eine Messung der Temperatur selbst zumindest an einem Teil der Schweißperle kann das Abkühlverhalten der Schweißperle unmittelbar beschreiben, so dass die Überwachung der Anbindungsfläche besonders genau und zuverlässig erfolgen kann. Allerdings ist die Messung der Temperatur apparativ relativ aufwändig.

Bevorzugt ist eine Weiterentwicklung dieser Variante, bei der die Temperatur mittels Quotientenpyrometrie gemessen wird, wobei die Intensitäten einer Glühemission zumindest des Teils der Schweißperle bei zwei unterschiedlichen Wellenlängen gemessen werden. Die Messung mittels Quotientenpyrometrie ist besonders zuverlässig. Hierfür können Photodioden mit schmalbandigen Bandpassfiltern, die für die beiden unterschiedlichen Wellenlängen selektiv sind, eingesetzt werden. Alternativ kann auch eine Thermografiekamera eingesetzt werden, oder ein gemessenen Spektrum kann an einen grauen Strahler (zB Kupfer) angefittet werden.

### Varianten betreffend den Parameter

Besonders bevorzugt ist eine Variante, bei der der Parameter eine Zeitdauer ist, die zwischen einem ersten definierten Zustand und einem zweiten definierten Zustand während des Abkühlens der Schweißperle vergeht. Eine Zeitdauer ist relativ einfach zu messen. Typischerweise wird zumindest einer der definierten Zustände durch den zeitlichen Verlauf der wenigstens einen Messvariable erkannt. Typische definierte Zustände sind das Erreichen von bestimmten Temperaturen oder erkannte Phasenübergänge.

Bei einer bevorzugten Weiterentwicklung dieser Variante ist der erste definierte Zustand das Ende der Einwirkung des Bearbeitungs-Laserstrahls. Der Zeitpunkt des Endes der Einwirkung des Bearbeitungs-Laserstrahls ("Abschalten") ist typischerweise über die Steuerung des Schweißprozesses vorgegebenen bzw. bekannt, und braucht daher nicht gesondert ermittelt zu werden.

Ebenso bevorzugt ist eine Weiterentwicklung, bei der der zweite definierte Zustand die vollständige Erstarrung der Schweißperle ist. Der Phasenübergang von flüssig nach fest kann in der Regel optisch einfach erkannt werden, und ist daher gut als zweiter definierter Zustand geeignet. Dabei ändert sich sowohl das Emissionsverhalten als auch die Reflektivität relativ stark. Zudem kann die (vollständige) Erstarrung optisch auch dadurch erkannt werden, dass Bewegungen an der Schweißperle vollständig zum Erliegen gekommen sind.

Bei einer vorteilhaften Weiterentwicklung wird der erste und/oder der zweite definierte Zustand dadurch erkannt, dass die Messvariable einen Schwellwert erreicht. Dieses Vorgehen ist besonders einfach. Insbesondere kann die vollständige Erstarrung der Schweißperle dadurch erkannt werden, dass die Intensität der Glühemission einen Schwellwert erreicht (bzw. nach unten durchbricht).

Bevorzugt ist auch eine Weiterentwicklung, bei der der erste und/oder der zweite definierte Zustand dadurch erkannt wird, dass eine erste und/oder zweite zeitliche Ableitung der Messvariable einen Schwellwert erreicht,
insbesondere dadurch, dass eine erste und/oder zweite zeitliche Ableitung der Messvariable den Schwellwert erreicht, nachdem zuvor die Messvariable über eine vorgegebene Mindestdauer nur innerhalb vorgegebener Grenzen geschwankt hat. In der ersten oder der zweiten Ableitung der Messvariablen sind Informationen über den Abkühlverlauf, insbesondere über Phasenübergänge, oftmals leichter erkennbar als in der Messvariablen selbst. Insbesondere führt die Erstarrung der Schweißperle zu einem Anstieg der Reflektivität für einen Beobachtungs-Lichtstrahl, der als positiver Gradient in der ersten Ableitung der Intensität der reflektierten Laserstrahlung leicht erkennbar ist.

Bevorzugt ist eine Variante, bei der der Parameter ein zeitlicher Gradient der Messvariable ist, der zu einem vorgegebenen Zeitpunkt oder gemittelt über einen vorgegebenen Zeitraum bestimmt wird. Der Gradient kann zum Beispiel unmittelbar nach dem Ende der Einwirkung des Bearbeitungs-Laserstrahls, oder gemittelt über einige zehn Millisekunden nach dem Ende des Bearbeitungszeitraums bestimmt werden. Die Messvariable kann dabei insbesondere eine Temperatur oder eine Intensität der Glühemission sein. Insbesondere kann der Gradient zur Temperatur oder zur Intensität der Glühemission bestimmt werden, wenn die Schweißperle noch vollständig flüssig ist.

### Varianten zur Auswertung des Parameters

Bei einer bevorzugten Variante des erfindungsgemäßen Verfahrens ist vorgesehen,
dass der Parameter oder eine aus dem Parameter quantitativ bestimmte Anbindungsfläche mit einem Entscheidungswert verglichen wird,
dass wenn der Entscheidungswert nicht erreicht wird, die Anbindungsfläche der Verschweißung als zu klein erkannt wird, insbesondere wobei in diesem Fall betroffene Stableiter aussortiert werden oder ein Nachschweißen vorgenommen wird,
und dass wenn der Entscheidungswert erreicht wird, die Anbindungsfläche als ausreichend groß erkannt wird und betroffene Stableiter für die weitere Verwendung zugelassen werden. Dieses Vorgehen ist besonders einfach.

Vorteilhaft ist eine Weiterentwicklung zu dieser Variante, die vorsieht, dass mittels einer Kamera ein nicht von der Schweißperle bedeckter Anteil eines Querschnitts der Stableiter bestimmt wird, und dass falls der nicht bedeckte Anteil des Querschnitts einen Grenzwert überschreitet, die Anbindungsfläche der Verschweißung der Stableiter als zu klein erkannt wird, auch wenn der Entscheidungswert erreicht wird. Falls die Schweißperle den Querschnitt der Stableiter unvollständig bedeckt, wird die gegenseitige Anbindungsfläche der Stableiter ausnahmsweise nicht über die Masse bzw. die Größe der Schweißperle ausreichend charakterisiert. Vielmehr ist anzunehmen, dass der Querschnitt, der die elektrische Leitung zwischen den Stableitern begrenzt, im Bereich des Kontakts der Schweißperle zum Stableiter liegt. Ist dieser zu klein, ist die Verschweißung genauso unbrauchbar wie im Falle einer zu kleinen Schweißperle. Mit dieser Weiterentwicklung wird die Zuverlässigkeit der Erkennung elektrisch unbrauchbarer Verschweißungen weiter erhöht.

Bevorzugt ist auch eine Variante, bei der eine Vielzahl von Paaren von Stableitern nacheinander verschweißt werden, wobei ein oder mehrere Schweißparameter beim Verschweißen der Paare von Stableitern in einem Regelkreis optimiert und/oder nachgeregelt werden, so dass der Parameter oder eine aus dem Parameter quantitativ bestimmte Anbindungsfläche für die Paare von verschweißten Stableitern auf einen vorgegebenen Zielwert eingestellt werden. Durch dieses Vorgehen lässt sich die Qualität der produzierten Verschweißungen erhöhen, und die Anzahl von Aussortiervorgängen und/oder Nachschweißvorgängen minimieren.

### Anlage zum Laserschweißen für das erfindungsgemäße Verfahren

In den Rahmen der vorliegenden Erfindung fällt auch eine im Anspruch 15 definierte Anlage zum Laserschweißen von kupferhaltigen, gebogenen Stableitern, insbesondere von Hairpins für einen Elektromotor,
mit einer Halteeinrichtung, mit der zwei Stableiter überlappend angeordnet werden können, insbesondere wobei die Halteeinrichtung einen Statorträger mit einer Vielzahl von zu verschweißenden Stableitern umfasst,
und mit einem Laserbearbeitungskopf, der einen Bearbeitungs-Laserstrahl bereitstellt, mit dem die zwei Stableiter miteinander verschweißt werden,
so dass sich eine Schweißperle ausbildet, durch die die Stableiter miteinander verbunden werden, wobei die Anlage weiterhin eine Sensoreinrichtung umfasst, mit der nach dem Ende der Einwirkung des Bearbeitungs-Laserstrahls während des Abkühlens der Schweißperle wenigstens eine mit der Temperatur der Schweißperle veränderliche Messvariable zumindest an einem Teil der Schweißperle messbar ist,
die dadurch gekennzeichnet ist,
dass die Anlage weiterhin eine elektronische Auswerteeinrichtung aufweist, die dazu eingerichtet ist, insbesondere dazu programmiert ist, aus der wenigstens einen gemessenen Messvariablen einen von der Wärmekapazität der Schweißperle abhängigen Parameter zu bestimmen,
und dass die elektronische Auswerteeinrichtung weiterhin dazu ausgebildet ist, insbesondere dazu programmiert ist, aus dem Parameter eine durch die Schweißperle eingerichtete Anbindungsfläche qualitativ oder quantitativ zu bestimmen. Die erfindungsgemäße Anlage ist eingerichtet zur Ausführung eines erfindungsgemäßen, oben beschriebenen Verfahrens.

Mit der Anlage ist es möglich, die Anbindungsfläche beim Laserschweißen von kupferhaltigen, gebogenen Stableitern zur Verfügung einfach, schnell und zerstörungsfrei zu überwachen, insbesondere auch online während der Fertigung der Verschweißungen.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1a: zeigt in einer schematischen Seitenansicht zwei gebogene Stableiter in teilweise überlappender Anordnung, die im Rahmen der Erfindung miteinander verschweißt werden sollen;
- Fig. 1b: zeigt in einer schematischen Schrägansicht die aneinander liegenden Endbereiche der beiden Stableiter von Fig. 1a, mit Blick auf die stirnseitigen Endflächen;
- Fig. 2: zeigt in einer schematischen Seitenansicht die Endbereiche von zwei gemäß der Erfindung verschweißten Stableitern, die über eine Schweißperle miteinander verbunden sind, mit Markierung der Anbindungsfläche;

- Fig. 3: zeigt ein Diagramm darstellend die röntgenographisch bestimmte Anbindungsfläche und die gemessene Erstarrungszeit für sechs beispielhafte verschweißte Messproben;
- Fig. 4: zeigt eine schematische Darstellung einer ersten Ausführungsform einer erfindungsgemäßen Anlage zum Laserschweißen, mit Messung der Intensität der Glühemission an einem Teil der Schweißperle mittels einer Kamera;
- Fig. 5a: zeigt in einer schematischen Seitenansicht die Endbereiche von zwei gemäß der Erfindung verschweißten Stableitern, die über eine Schweißperle miteinander verbunden sind, mit Markierung des von der Kamera der Anlage von Fig. 4 aufgenommenen Bildes und des für die Bestimmung der Glühemission ausgewählten Teilbereichs dieses Bildes, entsprechend einem zentralen, oberen Teil der Schweißperle;
- Fig. 5b: zeigt in einer schematischen Seitenansicht die Endbereiche von zwei gemäß der Erfindung verschweißten Stableitern, die über eine Schweißperle miteinander verbunden sind, mit Markierung des von einer alternativ angeordneten Kamera aufgenommenen Bildes und des für die Bestimmung der Glühemission ausgewählten Teilbereichs dieses Bildes, entsprechend einem seitlich an der Schweißperle liegenden Teils der Schweißperle;
- Fig. 6: zeigt ein schematisches Diagramm darstellend einen mittleren Grauwert, entsprechend der Intensität der Glühemission, der an einem Teil der Schweißperle während ihres Abkühlens als Funktion der Zeit beobachtet wird (oben), sowie die erste zeitliche Ableitung des Grauwerts (Mitte) und die zweite zeitliche Ableitung des Grauwerts (unten), für die Erfindung;
- Fig. 7: zeigt eine schematische Darstellung einer zweiten Ausführungsform einer erfindungsgemäßen Anlage zum Laserschweißen, mit Messung der Intensität eines an der Schweißperle reflektierten Beobachtungs-Laserstrahls mittels einer Kamera;

- Fig. 8: zeigt ein schematisches Diagramm darstellend einen mittleren Grauwert, entsprechend der Überlagerung der Intensität der Glühemission und der Intensität des reflektierten Beobachtungs-Laserstrahls, der an einem Teil der Schweißperle während ihres Abkühlens als Funktion der Zeit mit der Anlage von Fig. 7 beobachtet wird (oben), sowie die erste zeitliche Ableitung des Grauwerts (Mitte) und die zweite zeitliche Ableitung des Grauwerts (unten), für die Erfindung;
- Fig. 9: zeigt eine schematische Darstellung einer dritten Ausführungsform einer erfindungsgemäßen Anlage zum Laserschweißen, mit Messung der Temperatur der Schweißperle mittels Quotientenpyrometrie;
- Fig. 10: zeigt ein schematisches Diagramm darstellend die Temperatur, die an einem Teil der Schweißperle während ihres Abkühlens als Funktion der Zeit in der Anlage von Fig. 9 beobachtet wird (oben), sowie die erste zeitliche Ableitung der Temperatur (Mitte) und die zweite zeitliche Ableitung der Temperatur (unten), für die Erfindung;
- Fig. 11a: zeigt eine schematische Aufsicht auf die Schweißperle stirnseitig auf die Endbereiche zweier verschweißter Stableiter, wobei die Querschnitte der Endbereiche der Stableiter vollständig von der Schweißperle abgedeckt sind, für die Erfindung;
- Fig. 11b: zeigt eine schematische Aufsicht auf die Schweißperle stirnseitig auf die Endbereiche zweier verschweißter Stableiter, wobei die Querschnitte der Endbereiche der Stableiter nur teilweise von der Schweißperle abgedeckt sind, für die Erfindung.

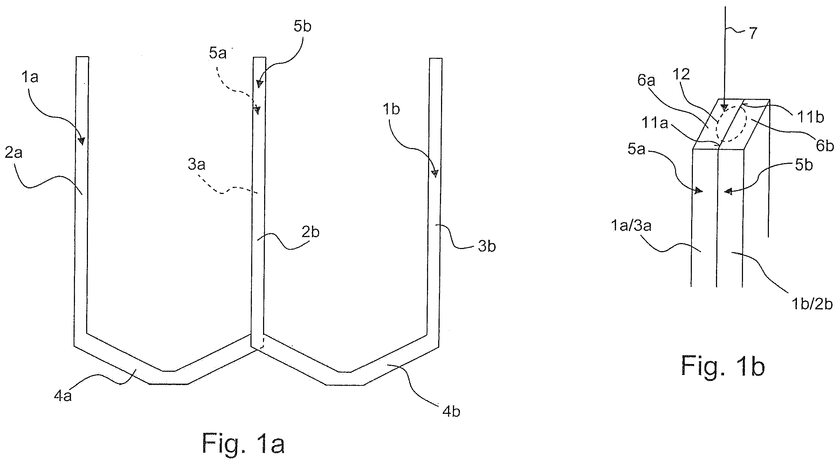

Die **Fig. 1a** zeigt in einer schematischen Seitenansicht zwei kupferhaltige, gebogene Stableiter 1a, 1b, ausgebildet als so genannte Hairpins, zur Fertigung einer elektrodynamischen Maschine, etwa eines Elektromotors. Jeder Stableiter 1a, 1b ist näherungsweise U-förmig ausgebildet und verfügt über zwei Beine (Schenkel) 2a, 3a und 2b, 3b sowie einen die Beine verbindenden Mittelteil 4a, 4b.

Die Stableiter 1a, 1b sollen elektrisch leitfähig miteinander verbunden werden, und sollen dafür an ihren Endbereichen 5a, 5b miteinander verschweißt werden. Zu diesem Zweck sind das Bein 3a des ersten Stableiters 1a und das Bein 2b des zweiten Stableiters 1b überlappend und hier auch aneinander anliegend angeordnet.

Wie in **Fig. 1b** in schematischer Schrägansicht auf die Endbereiche 5a, 5b ersichtlich, sind dabei die stirnseitigen Endflächen 6a, 6b der beiden Stableiter 1a, 1b auf ungefähr gleicher Höhe angeordnet worden, und die langen Seiten 11a, 11b der Endbereiche 5a, 5b der Beine 3a, 2b liegen flächig und fluchtend aneinander an, wobei die Beine 3a, 2b in nicht näher dargestellter Weise aneinandergepresst werden. Die Beine 3a, 2b sind parallel zueinander und vertikal ausgerichtet, sodass die beiden Endflächen 6a, 6b nach oben ausgerichtet sind.

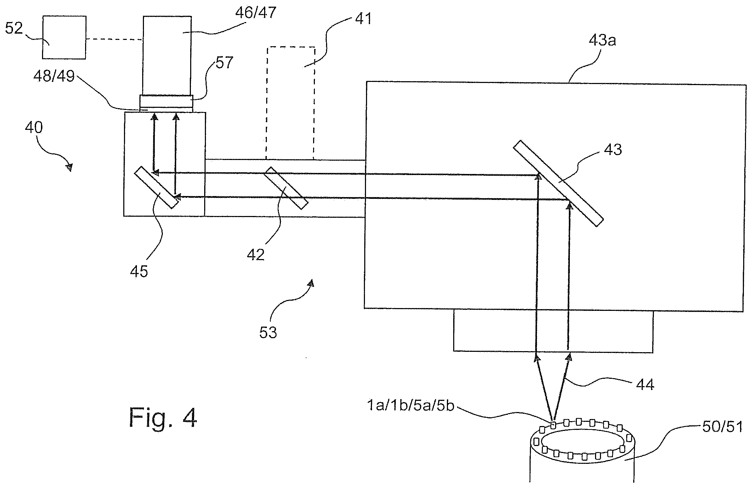

Zum Verschweißen der beiden Endbereiche 5a, 5b wird ein Bearbeitungs-Laserstrahl 7 eingesetzt, der hier die stirnseitigen Endflächen 6a, 6b in einer sich wiederholenden Kreisbahn 12 überstreicht. Der Bearbeitungs-Laserstrahl 7 trifft dabei näherungsweise senkrecht auf die Endflächen 6a, 6b; man beachte dabei, dass der Einfallswinkel des Bearbeitungs-Laserstahls 7 während der Fertigung verschiedener Paare von Stableitern typischerweise etwas variiert, um die meist in einem Statorträger (nicht näher dargestellt, vgl. aber z.B. Fig. 4) angeordneten Stableiter 1a, 1b nicht zu oft versetzen zu müssen. Typischerweise weicht der Bearbeitungs-Laserstrahl 7 dabei nicht mehr als 40° von einem senkrechten Einfall ab.

Durch die Einwirkung des Bearbeitungs-Laserstrahls 7 schmilzt das Material der Stableiter 1a, 1b nahe den Endflächen 6a, 6b auf, und es bildet sich eine so genannte Schweißperle. Man beachte, dass dabei in der Regel eine vorgegebene Laserleistung für eine vorgegebenen Zeit für eine Vielzahl von Paaren von zu verschweißenden Stableitern eingesetzt wird, deren Anbindungsfläche im Rahmen der Erfindung überwacht werden soll.

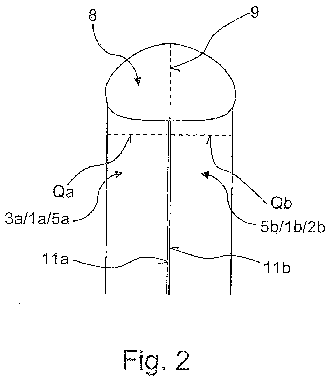

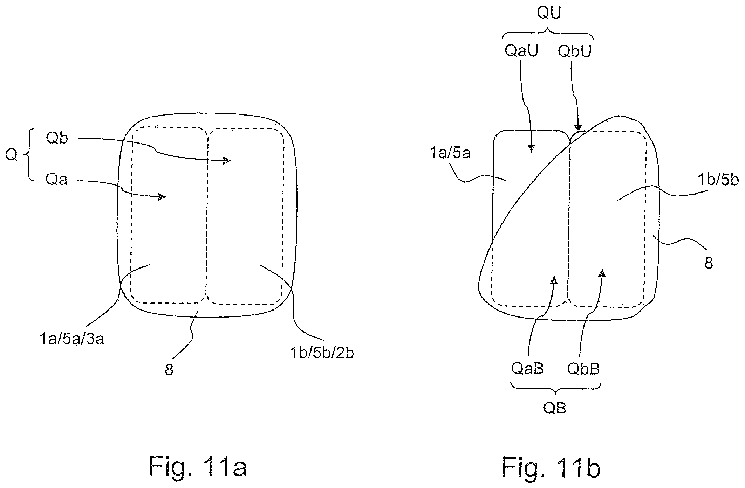

Die **Fig. 2** zeigt die Endbereiche 5a, 5b der Stableiter 1a, 1b nach dem Laserschweißen. Die Stableiter 1a, 1b sind über die Schweißperle 8 elektrisch leitfähig miteinander verbunden. Die Schweißperle 8 sitzt dabei in der Regel vollflächig auf beiden Stableitern 1a, 1b auf, bedeckt also jeweils deren volle Querschnittsflächen Qa, Qb (näheres dazu siehe auch Fig. 11a später).

Die Qualität der elektrisch leitfähigen Verbindung der beiden Stableiter 1a, 1b wird durch die so genannte Anbindungsfläche 9 wesentlich bestimmt. Dies ist die Querschnittsfläche, die durch die Schweißperle 8 für eine elektrische Stromleitung vom ersten Stableiter 1a zum zweiten Stableiter 1b zur Verfügung gestellt wird, und entspricht näherungsweise der Schnittfläche der Schweißperle 8 in der Kontaktebene der aneinander anliegenden, langen Seiten 11a, 11b der Beine 3a, 2b der Stableiter 1a, 1b.

Im Allgemeinen ist die Anbindungsfläche 9 umso größer, je größer die Schweißperle 8 ist. Die Größe der Schweißperle 8 (sei es ihre Höhe H, oder auch ihr Volumen) ist aufgrund von häufigen Unregelmäßigkeiten an den Endbereichen 5a, 5b der Stableiter 1a, 1b (etwa ungleichmäßige oder schräge Einkürzungen bereits vor Beginn des Schweißprozesses) sehr schwierig zu bestimmen, und insbesondere ist es nach den Erfahrungen der Erfinder über direkte Bildauswertungen kaum möglich, eine zuverlässige Bestimmung der Größe der Schweißperle 8 vorzunehmen.

Daher sieht die vorliegende Erfindung vor, die Größe der Schweißperle 8 indirekt zu bestimmen, indem der Verlauf der Abkühlung der Schweißperle 8 nach dem Abschalten des Bearbeitungs-Laserstrahls verfolgt wird. Bei Abschalten des Bearbeitungs-Laserstrahls befindet sich typischerweise die gesamte Schweißperle 8 näherungsweise auf der Schmelztemperatur T_{S} des Materials der Stableiter 1a, 1b (Man beachte, dass der Bearbeitungs-Laserstrahl in gewissem Umfang Material der Stableiter 1a, 1b verdampft). Die Wärmeableitung aus der Schweißperle 8 erfolgt anschließend praktisch ausschließlich über die beiden Stableiter 1a, 1b und ist damit durch die Wärmeleitfähigkeit des Materials der Stableiter und deren zur Verfügung stehenden Querschnittsflächen Qa, Qb begrenzt ("eindimensionale Wärmeableitung"). Je größer die Schweißperle 8, desto mehr Wärmeenergie muss über die Stableiter 1a, 1b abgeleitet werden. Bei begrenzter Wärmeableitung führt dies dazu, dass die Temperatur einer großen Schweißperle 8 über die-Zeit langsamer absinkt als die Temperatur einer kleineren Schweißperle 8.

Die Erfindung sieht deshalb vor, eine von der Temperatur der Schweißperle 8 (oder eines Teils der Schweißperle 8) abhängige Messvariable als Funktion der Zeit zu messen, und aus dieser Messvariablen einen von der (absoluten) Wärmekapazität der Schweißperle abhängigen Parameter zu bestimmen. Die (absolute) Wärmekapazität der Schweißperle 8 ist proportional zu deren Masse und damit zu deren Volumen bzw. Größe. Entsprechend kann aus dem Parameter sodann qualitativ oder quantitativ auf die Anbindungsfläche geschlossen werden.

Die Messung einer temperaturabhängigen Messvariablen ist relativ einfach möglich, insbesondere auch optisch. Besonders einfach ist beispielsweise die Messung der Intensität der Glühemission der Schweißperle mit einer Kamera oder einer Photodiode möglich; ebenso kann als Messvariable auch die Temperatur zumindest einen Teils der Schweißperle diesen. Ein besonders einfach zu bestimmender, von der Wärmekapazität der Schweißperle 8 abhängiger Parameter ist die Zeitdauer zwischen dem Abschalten des Bearbeitungs-Laserstrahls und der (vollständigen) Erstarrung der Schweißperle ("Erstarrungszeit"); ebenso kann als Parameter ein Temperaturgradient verwendet werden.

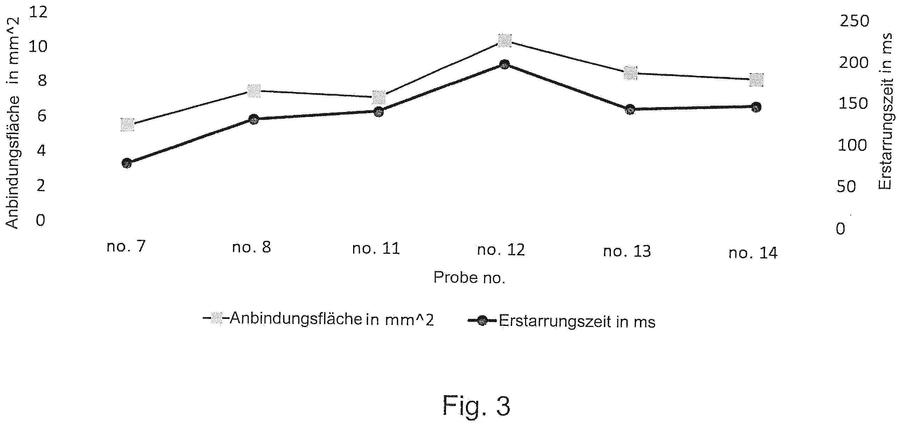

Die **Fig. 3** illustriert in einem Diagramm für verschiedene experimentelle Messproben, bei denen eine Verschweißung von Endbereichen von Stableiter-Dummies mit einem Bearbeitungs-Laserstrahl entsprechend der in Fig. 1b und Fig. 2 gezeigten Geometrie für die Erfindung vorgenommen wurde, jeweils eine über optische Beobachtungen bestimmte Erstarrungszeit (vgl. Kreis-Markierungen) und eine über herkömmliche Röntgenanalyse bestimmte tatsächliche Anbindungsfläche (vgl. Quadrat-Markierungen). Es ist deutlich zu erkennen, dass die Anbindungsfläche mit der Erstarrungsfläche stark korreliert. Entsprechend kann über die Erstarrungszeit auf die Anbindungsfläche rückgeschlossen werden, und diese qualitativ oder quantitativ bestimmt werden.

Im Diagramm von Fig. 3 kann ersehen werden, dass ab einer Erstarrungszeit von ca. 140 ms eine Anbindungsfläche von ca. 8 mm² erreicht wird. Wenn beispielsweise in dieser Situation im Rahmen der Überwachung der Anbindungsfläche der Stableiterverschweißungen eine Anbindungsfläche von wenigstens 8 mm² sichergestellt werden soll, kann aus einer Erstarrungszeit kürzer als 140 ms ("Entscheidungswert") auf eine unzureichende Anbindungsfläche kleiner 8 mm² geschlossen werden, und ab einer Erstarrungszeit von 140 ms auf eine ausreichende Anbindungsfläche von 8 mm² oder mehr. Dies wäre eine typische qualitative Bestimmung der Anbindungsfläche. Verschweißungen mit als zu klein erkannter Anbindungsfläche werden typischerweise einer Nachschweißung zugeführt.

Aus dem Diagramm kann auch, wenn gewünscht, beispielsweise ein einfacher, linearer funktionaler Zusammenhang zwischen der Erstarrungszeit und der Anbindungsfläche erhalten werden. In guter Näherung gilt hier für die Erstarrungszeit EZ und die Anbindungsfläche AF: $AF \left(EZ\right) = 0 , 0419 {mm}^{2} / ms * EZ + 2 , 426 {mm}^{2}$

Über einen solchen funktionalen Zusammenhang kann auch leicht eine quantitative Aussage über die Anbindungsfläche aus der Erstarrungszeit als Parameter getroffen werden.

Für die qualitative oder quantitative Bestimmung der Anbindungsfläche aus dem Parameter sind für eine jeweilige Schweißsituation (insbesondere Größe, Anordnung und Material der Stableiter) Kalibriermessungen nötig, wie sie in Fig. 3 gezeigt sind.

Falls gewünscht, können auf Basis einer quantitativen Bestimmung der Anbindungsfläche auch Schweißparameter (wie die Laserleistung oder die Dauer der Einwirkung des Bearbeitungs-Laserstrahls) auf eine bestimmte Anbindungsfläche optimiert bzw. eingeregelt werden.

Die **Fig. 4** zeigt in einer Schemadarstellung den Aufbau einer ersten erfindungsgemäßen Anlage 40 zum Laserschweißen, mit der das erfindungsgemäße Verfahren durchgeführt werden kann.

Die Anlage 40 verfügt über einen Bearbeitungs-Laser 41, mit dem über einen halbdurchlässigen Spiegel 42 ein Bearbeitungs-Laserstrahl (nicht näher dargestellt, vgl. aber hierzu Fig. 1b) mit einer Wellenlänge von hier 1030 nm über einen Scanner-Spiegel 43 einer programmierbaren Fokussier-Optik 43a auf die Endbereiche 5a, 5b eines Paars von Stableitern 1a, 1b gerichtet werden kann. Die Stableiter 1a, 1b sind in einer Halteeinrichtung 50 angeordnet, die hier als Statorträger 51 für einen Elektromotor ausgebildet ist; die Halteeinrichtung 50 hält typischerweise gleichzeitig mehr als dreißig Paare von Stableitern gleichzeitig.

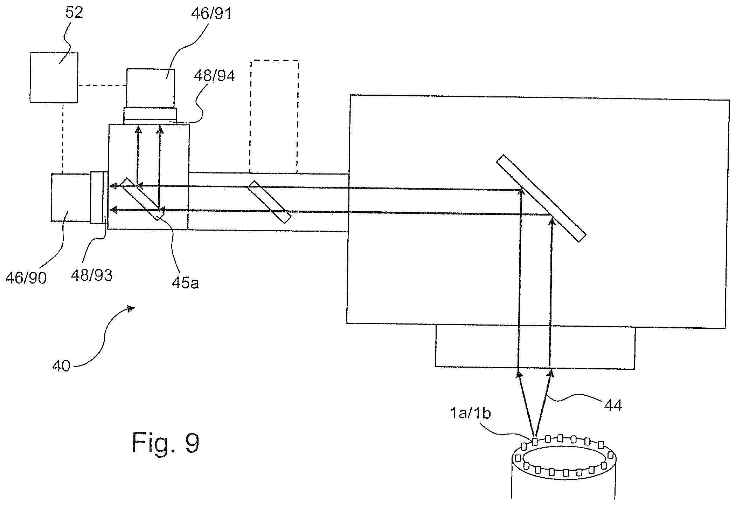

Nach Abschalten des Bearbeitungs-Laserstrahls zeigt die entstandene Schweißperle (nicht näher dargestellt, vgl. aber Fig. 2 hierzu) eine Glühemission 44. Die Glühemission 44 wird über den Scanner-Spiegel 43, den halbdurchlässigen Spiegel 42 und den Spiegel 45 sowie die Kollimationslinse 57 in eine Sensoreinrichtung 46, hier eine Kamera 47, abgebildet; alternativ könnte als Sensoreinrichtung 46 auch eine Photodiode eingesetzt werden (nicht näher dargestellt, vgl. aber Fig. 9). Vor der Kamera 47 ist ein optischer Filter 48 angeordnet, hier ein Bandpassfilter 49, der nur den Spektralbereich von 800 nm bis 1000 nm passieren lässt. Die Kamera 47 ist an eine elektronische Auswerteeinrichtung 52 angeschlossen. Die Kamera 47 blickt hier gemäß dem aufgezeigten Strahlengang von oben auf die Endbereiche 5a, 5b der Stableiter 1a, 1b bzw. die dortige Schweißperle.

Die programmierbare Fokussieroptik 43a sowie die Spiegel 42, 45 und die Kamera 47 sind hier zu einem Laserbearbeitungskopf 53 zusammengefasst; man beachte aber, dass die Kamera auch unabhängig vom Laserbearbeitungskopf angeordnet werden kann, insbesondere um seitlich auf die Schweißperle zu blicken.

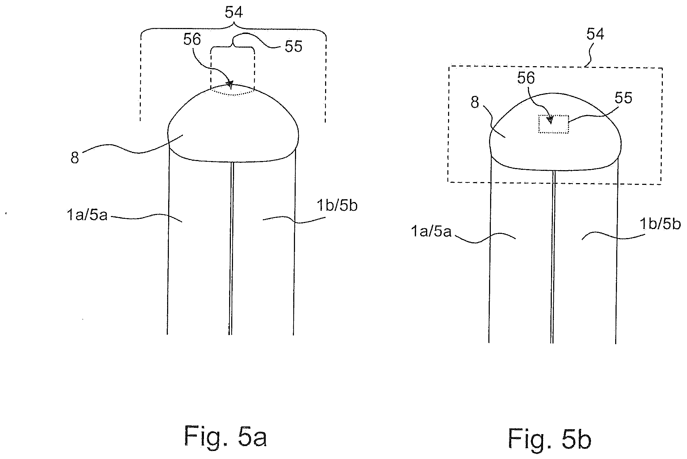

Mit der Kamera 47 wird hier von oben ein Bild 54 von der Schweißperle 8 aufgenommen, wie in **Fig. 5a** schematisch dargestellt. Von einem Teilbereich 55 des Bildes 54 wird ein mittlerer Grauwert (mittlere Helligkeit) als Messvariable bestimmt; dieser Grauwert entspricht der Intensität der Glühemission der Schweißperle 8 in einem dem Teilbereich 55 entsprechenden Teil 56 der Schweißperle 8, wobei dieser Teil 56 hier zentral und oben an der Schweißperle 8 liegt. Der Teil 56 liegt somit gegenüber den vormaligen Teilflächen (stirnseitige Endflächen), auf die der Bearbeitungs-Laserstrahl zuvor gefallen ist (vgl. Fig. 1b hierzu).

Alternativ kann auch die Kamera seitlich auf die Schweißperle 8 gerichtet sein, wie in **Fig. 5b** dargestellt. Der Teilbereich 55 des entsprechenden Bildes 54 wird dann typischerweise entfernt vom Rand der Schweißperle 8, ungefähr in der Mitte der Schweißperle 55, gewählt.

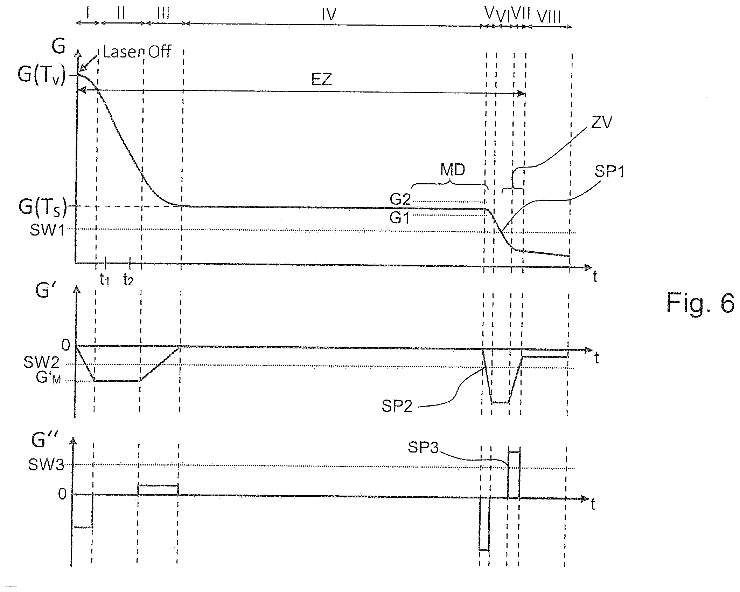

Die **Fig. 6** zeigt oben in einem schematischen Diagramm einen typischen, mit der Anlage von Fig. 4 beobachteten Verlauf des mittleren Grauwerts G als Funktion der Zeit t während der Abkühlung der Schweißperle. Der Grauwert G entspricht der Intensität der Glühemission der Schweißperle. Die Messung des mittleren Grauwerts erfolgt vom Abschalten des Bearbeitungs-Lasers bis nach dem Phasenübergang flüssig nach fest der Schweißperle.

Unmittelbar bei Abschaltung des Bearbeitungs-Laserstrahls wird ein Grauwert G(T_{V}) entsprechend der Verdampfungstemperatur T_{V} des Materials der Stableiter gemessen. Nach dem Abschalten des Bearbeitungs-Lasers (markiert mit "Laser off") fällt über die zeitlichen Abschnitte I, II, III der Grauwert G entsprechend dem Temperaturrückgang der noch vollständig flüssigen Schweißperle ab, und verharrt dann während einer Phase der von unten nach oben fortschreitenden Rekristallisation (Erstarrung) der Schweißperle im Abschnitt IV, bei einem nahezu konstanten Wert G(T_{S}) entsprechend der Schmelztemperatur T_{S} des Materials der Stableiter. In Abschnitt IV wird der Grauwert G an einem nach wie vor flüssigen Teil der Schweißperle bestimmt. In den Abschnitten V, VI und VII erfolgt die Erstarrung des letzten, noch flüssigen und hier beobachteten oberen Teils der Schweißperle, wobei der Grauwert deutlich abfällt, da durch den Phasenübergang von flüssig nach fest die Glühemission stark reduziert wird. Während des Abschnitts VI wandert die Erstarrungsfront durch den ausgewerteten Teilbereich des Bildes der Kamera, wobei hier G seinen steilsten und näherungsweise linearen Verlauf hat. In einem sich anschließenden Abschnitt VIII fällt dann der Grauwert G weiter, jedoch deutlich langsamer, entsprechend dem allmählichen weiteren Absinken der Temperatur der vollständig erstarrten Schweißperle.

Über den Schnittpunkt SP1 der Kurve des Grauwerts G(t) mit der Linie eines ersten Schwellwerts SW1, der bevorzugt einem Grauwert aus dem (erwarteten) Wertebereich des Abschnitts VI entspricht, kann die tatsächliche Erstarrungszeit EZ (entsprechend der Zeitdauer von Beginn des Abschnitts I bis zum Ende des Abschnitts VII) auf einfache Weise in guter Näherung bestimmt werden, etwa mit der elektronischen Auswerteeinheit (man beachte, dass aufgrund des flachen Verlaufs im Bereich der Abschnitte VII und VIII die Bestimmung eines Schnittpunkt dort deutlich größere Fehler aufwiese). Man beachte weiterhin, dass die Abschnitte V, VI und VII typischerweise kurz sind im Vergleich zu den Abschnitten I, II, III und IV. Falls gewünscht, kann ein bekannter (erwarteter) zeitlicher Versatz ZV zwischen dem Schnittpunk SP1 und dem Ende der Erstarrung auf den Zeitpunkt des festgestellten Schnittpunkts SP1 aufaddiert werden, um die tatsächliche Erstarrungszeit EZ noch besser anzunähern.

Für eine andere Feststellung der Erstarrungszeit EZ kann auf die erste zeitliche Ableitung G' des Grauwerts G zurückgegriffen werden, vgl. mittleres Diagramm von Fig. 6; die Ableitung kann numerisch erfolgen. In der ersten Ableitung G' ist der Abfall der Glühemission während der Erstarrung der Schweißperle in den Abschnitten V, VI und VII noch besser zu erkennen als in der Kurve von G; die Erstarrung ist im Abschnitt V durch ein negativ werdendes G' gut zu erkennen. Da jedoch auch zu Beginn der Abkühlung, etwa im Abschnitt I bereits ähnliche (negative) Werte von G' vorkommen, wird die Erstarrungszeit EZ näherungsweise durch einen Schnittpunkt SP2 der Kurve von G' mit der Linie eines zweiten Schwellwerts SW2 kurz unter "null" bestimmt, bezüglich dessen zuvor die Kurve von G über eine Mindestdauer MD nur innerhalb vorgegebener Grenzen G1, G2 geschwankt hat. Letzteres Kriterium ist nur vor dem Schnittpunkt SP2 im Abschnitt IV (wo G' näherungsweise "null" ist) und zu Beginn des Abschnitts V gegeben (und nicht etwa in den Abschnitten I, II und VII), so dass dadurch der zweite Schnittpunkt SP2 eindeutig feststellbar ist. Man beachte wiederum, dass die Abschnitte V, VI und VII kurz sind im Vergleich zu den Abschnitten I, II, III und IV; falls gewünscht, kann wiederum ein bekannter (erwarteter) zeitlicher Versatz zwischen dem Schnittpunkt SP2 und dem Ende der Erstarrung auf den Zeitpunkt des Schnittpunkts SP1 aufaddiert werden, um die Erstarrungszeit EZ noch genauer zu bestimmen (siehe oben).

Eine etwas genauere Bestimmung der Erstarrungszeit EZ ist auch auf Basis der zweiten zeitlichen Ableitung G" des Grauwerts G möglich, vgl. unteres Diagramm in Fig. 6; die zweite zeitliche Ableitung kann ebenfalls numerisch erfolgen, etwa in der elektronischen Auswerteeinheit. Hier wird die vollständige Erstarrung recht genau durch einen Schnittpunkt SP3 der Kurve von G" mit der Linie eines dritten Schwellwerts SW3 bestimmt, der deutlich über "null" so groß gewählt ist, dass dieser Schwellwert SW3 erstmals im Abschnitt VII (und nicht etwa bereits im Abschnitt III) erreicht wird. Nötigenfalls kann auch hier eine Absicherung über eine zuvor vergangene Mindestdauer erfolgen, innerhalb der der Grauwert G nur innerhalb vorgegebener Grenzen geschwankt hat, und es kann wiederum ein bekannter (erwarteter) zeitlicher Versatz zwischen dem festgestellten Schnittpunkt SP3 und dem tatsächlichen Ende der Erstarrung aufaddiert werden, um EZ genauer zu bestimmen (jeweils nicht näher dargestellt, aber siehe oben).

Als von der absoluten Wärmekapazität abhängiger Parameter eignet sich (neben der Erstarrungszeit EZ) auch gut der mittlere (zeitliche) Gradient G'_{M} des Grauwerts G in einem vorgegebenen Zeitraum zwischen zwei Zeitpunkten t1 und t2, die bevorzugt im Abschnitt II gewählt sind. In diesem Abschnitt II ist der Absolutbetrag von G' relativ groß und näherungsweise konstant und daher gut zu bestimmen.

Man beachte, dass die Kurven für G, G' und G" hier idealisiert dargestellt sind, und in der Praxis zeitlich in der Regel und den aufgezeigten Verlauf etwas schwanken; durch eine ausreichend große Integrationsfläche (Teilfläche des von der Kamera beobachteten Bildes) kann jedoch eine gute Glättung der Kurven erreicht werden.

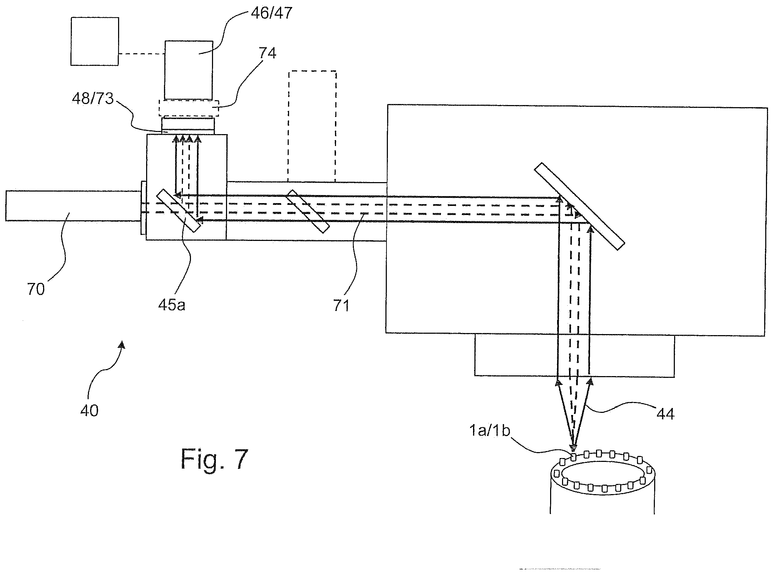

Die **Fig. 7** zeigt in einer Schemadarstellung den Aufbau einer zweiten erfindungsgemäßen Anlage 40 zum Laserschweißen, mit der das erfindungsgemäße Verfahren durchgeführt werden kann. Es werden nur die wesentlichen Unterschiede zur Anlage von Fig. 4 erläutert.

Bei der Anlage 40 von Fig. 7 wird während der Abkühlung der Schweißperle an den Stableitern 1a, 1b die Schweißperle mit einem Beobachtungs-Lichtstrahl 71, hier Beobachtungs-Laserstrahl, einer (mittleren) Wellenlänge von hier 810 nm aus einer Beobachtungs-Lichtquelle 70, hier einem Beobachtungs-Laser, durch einen halbdurchlässigen Spiegel 45a beleuchtet. Die Beobachtungs-Lichtquelle 70 bringt keine merkliche Energie in die Stableiter 1a, 1b ein, jedoch wird der Beobachtungs-Laserstrahl 71 an der Schweißperle abhängig von der Temperatur der Schweißperle reflektiert. Die Intensität des an der Schweißperle reflektierten Beobachtungs-Lichtstrahls 71 wird in der Kamera 47 registriert, zusammen mit der Glühemission 44 der Schweißperle.

Um den Anteil der Glühemission 44 an der gemessenen Intensität bzw. dem gemessenen Grauwert an der Kamera 47 zu minimieren, ist vor der Kamera 47 ein schmalbandiger Bandpassfilter 73 als optischer Filter 48 angeordnet, der hier lediglich Wellenlängen im Bereich 800 nm bis 820 nm passieren lässt.

Optional kann zusätzlich ein Polarisationsfilter 74 vor der Kamera 47 vorgesehen sein, mit dem der Kontrast im von der Kamera 47 aufgenommenen Bild verbessert werden kann.

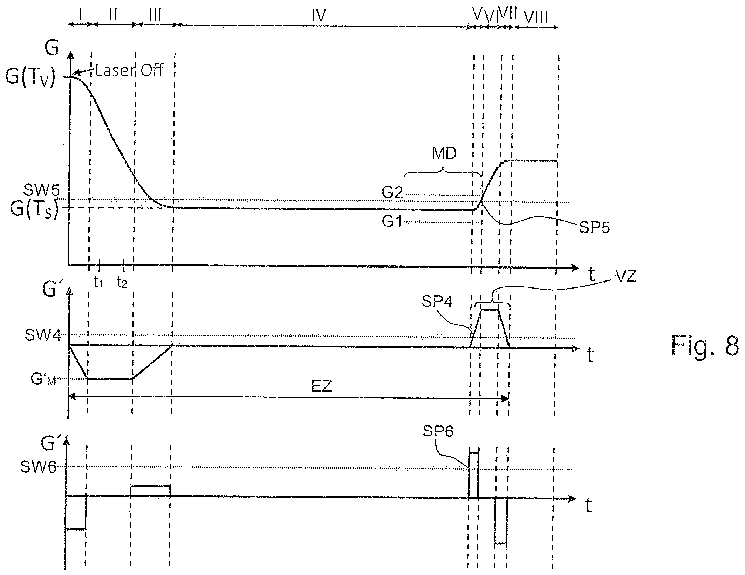

Die **Fig.** 8 zeigt oben in einem schematischen Diagramm einen typischen, mit der Anlage von Fig. 7 beobachteten Verlauf des mittleren Grauwerts G als Funktion der Zeit t während der Abkühlung der Schweißperle. Der Grauwert G entspricht der Überlagerung der Intensität der Glühemission der Schweißperle und der Intensität des reflektierten Beobachtungs-Laserstrahls. Es werden nur die wesentlichen Unterschiede zur Fig. 6 erläutert.

In den ersten Abschnitten I, II und III dominiert die Glühemission die an der Kamera gemessene Intensität, und entspricht daher weitgehend dem Verlauf in Fig. 6: Der Grauwert G nimmt entsprechend der mit der Temperatur abnehmenden Glühemission der flüssigen Schweißperle ab. Im Abschnitt IV bleibt der Grauwert G wiederum konstant.

Ab dem Abschnitt IV dominiert jedoch die Intensität des an der Schweißperle reflektierten Beobachtungs-Laserstrahls den Grauwert. Mit dem Phasenübergang ab Abschnitt V von flüssig nach fest steigt die Reflektivität der Schweißperle stark an (bzw. die Absorption nimmt stark ab), und entsprechend steigt der Grauwert G an. In Abschnitt VIII, nach dem vollständigen Erstarren der Schweißperle, bleibt die Intensität des reflektierten Laserlichts näherungsweise gleich.

Dies ist am besten über die erste zeitliche Ableitung G' des Grauwerts G festzustellen, vgl. das mittlere Diagramm der Fig. 8. Wenn die Kurve von G' die Linie eines Schwellwert SW4, der geringfügig über null gewählt ist, erstmalig schneidet, entspricht der Schnittpunkt SP4 in guter Näherung der Erstarrungszeit EZ; falls gewünscht, kann ein bekannter (erwarteter) zeitlicher Versatz ZV zwischen dem Zeitpunkt des Schnittpunkts SP4 und dem Ende der Erstarrung (am Ende von Abschnitt VII) auf den festgestellten Zeitpunkt des Schnittpunkts SP4 aufaddiert werden, um EZ noch genauer zu bestimmen.

Alternativ ist es auch möglich, das Erreichen von des Schwellwerts SW5 (bei Schnittpunkt SP5) für den Grauwert G oder des Schwellwerts SW6 (bei Schnittpunkt SP6) für die zweite Ableitung G" des Grauwerts G (vgl. unteres Diagramm) zur Bestimmung der Erstarrungszeit EZ vorzusehen, die jedoch über eine davorliegende Mindestdauer MD, in der der Grauwert G lediglich innerhalb vorgegebener Grenzen G1, G2 variiert, abgesichert werden sollten (beispielshaft eingezeichnet bei G im oberen Diagramm, und den Schnittpunkt SP5 abzusichern).

Die **Fig. 9** zeigt in einer Schemadarstellung den Aufbau einer dritten erfindungsgemäßen Anlage 40 zum Laserschweißen, mit der das erfindungsgemäße Verfahren durchgeführt werden kann. Es werden wiederum nur die wesentlichen Unterschiede zur Anlage von Fig. 4 erläutert.

Die Anlage 40 ist für eine Quotientenpyrometrie eingerichtet, und verfügt entsprechend über zwei Sensoreinrichtungen 46, hier ausgebildet als zwei Photodioden 90, 91, die von der Glühemission 44 der Schweißperle an den Stableitern 1a, 1b beleuchtet werden. Beide Photodioden 90, 91 sind an die elektronische Auswerteeinrichtung 52 angeschlossen.

Vor der Photodiode 90 ist ein optischer Filter 48 angeordnet, der als schmalbandinger Bandpassfilter 93 ausgebildet ist und lediglich Licht einer ersten mittleren Wellenlänge λ1, einschließlich eines umgebenden kleinen Wellenlängenintervalls von typischerweise +/- 30 nm oder weniger, bevorzugt +/- 20 nm, durchlässt. Vor der Photodiode 91 ist ein optischer Filter 48 angeordnet, der ebenfalls als schmalbandiger Bandpassfilter 94 ausgebildet ist und lediglich Licht einer zweiten mittleren Wellenlänge λ2, einschließlich eines umgebenden kleinen Wellenlängenintervalls von typischerweise +/- 30 nm oder weniger, bevorzugt +/- 20 nm, durchlässt. Beispielsweise liegt λ1 bei 1550 nm und λ2 bei 1620 nm. Aus dem Verhältnis der Intensitäten bei den beiden Wellenlängen λ1 und λ2 kann die Temperatur der Schweißperle (bzw. in derem beobachteten Teil) als Messvariable bestimmt werden.

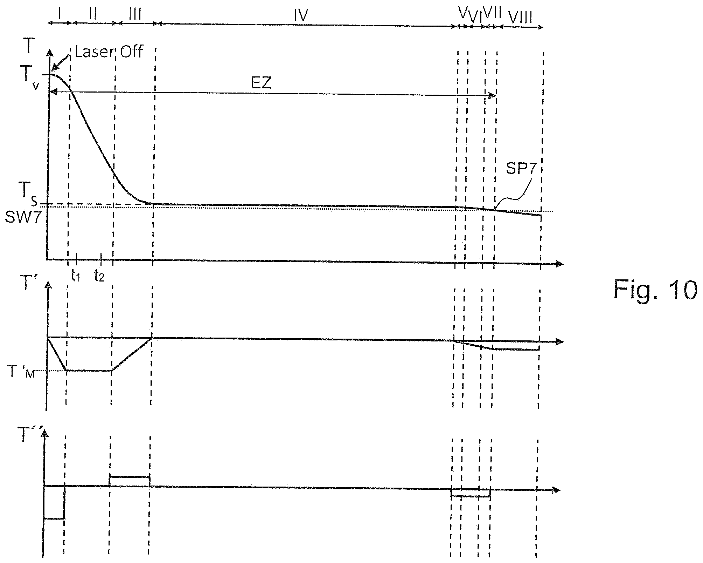

Die **Fig. 10** zeigt oben in einem schematischen Diagramm einen typischen, mit der Anlage von Fig. 9 beobachteten Verlauf der Temperatur T als Funktion der Zeit t während der Abkühlung der Schweißperle; die Temperatur wird dabei in einem zentralen, oberer Teil der Schweißperle beobachtet. Die Temperatur T verläuft weitgehend analog zum Grauwert der Glühemission, wie in Fig. 6 erläutert, insbesondere mit einem starken Temperaturabfall zu Beginn in den Abschnitten I, II und III, und mit einer über längere Zeit konstanten Temperatur T entsprechend der Schmelztemperatur T_{S} in Abschnitt IV, so dass eine Auswertung der Temperatur T insoweit analog zur Auswertung des Grauwerts der Glühemission erfolgen kann (siehe dort), weshalb hier von einer Wiederholung abgesehen wird. Man beachte aber, dass während der Erstarrung des letzten, am längsten flüssigen, hier beobachteten oberen Teils der Schweißperle in den Abschnitten V, VI VII die (im beobachteten Teilbereich mittlere) Temperatur nahezu konstant bleibt und sich nur sehr geringfügig ändert; erst nach der vollständigen Erstarrung der Schweißperle am Ende von Abschnitt VII fällt die Temperatur wieder langsam, mit einem näherungsweise konstanten Gradienten T ' ab. Bevorzugt wird hierbei ein Schwellwert SW7 für die Temperatur T angewandt, der kurz unter der Schmelztemperatur T_{S} (etwa am Anfang des erwarteten Abschnitt VIII) liegt, um als Parameter die Erstarrungszeit EZ in sehr guter Näherung über den Zeitpunkt des Schnittpunkts SP7 zu bestimmten. Ebenfalls gut möglich ist die Bestimmung des mittleren Temperaturgradienten T'_{M} zwischen den Zeitpunkten t1 und t2 im Abschnitt II als Parameter.

Beim Laserschweißen der Endbereiche 5a, 5b der Stableiter 1a, 1b werden in der Regel die Querschnitte Qa, Qb der Stableiter 1a, 1b (senkrecht zur Erstreckungsrichtung der Beine 3a, 2b) von der Schweißperle 8 vollständig abgedeckt, wie in **Fig. 11a** schematisch in Aufsicht illustriert.

In seltenen Fällen kommt es jedoch vor, etwa infolge von Spritzerbildung, dass die Schweißperle 8 bei einem der Stableiter 1a, 1b oder auch beiden Stableitern 1a, 1b deren Querschnitt nur unvollständig bedeckt, wie in **Fig. 11b** dargestellt. Im gezeigten Beispiel ist für den Stableiter 1a etwa 1/3 des Querschnitts Qa ein unbedeckter Anteil QaU, und etwa 2/3 des Querschnitts Qa ein von der Schweißperle 8 bedeckter Anteil QaB. Für den Stableiter 1b ist etwa 1/50 des Querschnitts Qb ein unbedeckter Anteil QbU, und etwa 49/50 des Querschnitts Qb ein von der Schweißperle 8 bedeckter Anteil QbB, was kaum ins Gewicht fällt. Insgesamt ist hier ca. 1/6 des gesamten Querschnitts Q=Qa+Qb der Stableiter 1a, 1b unbedeckt, entsprechend einem unbedeckter Anteil QU des gesamten Querschnitts Q von ca. 1/6.

Im Rahmen der Erfindung kann vorgesehen sein, die Anbindungsfläche auch dann als unzureichend einzustufen, wenn unabhängig von der Beobachtung der Messvariablen und dem daraus bestimmten Parameter der unbedeckte Anteil QU von Q einen Grenzwert GW überschreitet. Bevorzugt wird der Grenzwert GW zu 0,10 oder weniger, bevorzugt 0,05 oder weniger, bestimmt, bezogen auf den vollen Querschnitt Q. Dadurch kann eine schlechte elektrische Verbindung zwischen den Stableitern 1a, 1b erkannt und ggf. durch Nachschweißen beseitigt werden.

Zu geringe, von der Schweißperle bedeckte Anteile QB des gesamten Querschnitts Q der Stableiter 1a, 1b sind auch deshalb tückisch, weil sie zu einer verschlechterten Wärmeableitung aus der Schweißperle 8 führen. Die verlangsamte Abkühlung der Schweißperle 8 täuscht eine größere Schweißperle 8 und entsprechend eine ausreichend große Anbindungsfläche bei erfindungsgemäßen Beobachtung der temperaturabhängigen Messvariablen und des daraus bestimmten Parameters vor, obwohl die Anbindungsfläche tatsächlich zu klein ist. Durch die zusätzliche Bestimmung des unbedeckten Anteils QU, die durch eine optische Auswertung einer Aufsicht auf die Schweißperle leicht möglich ist, kann diese Problematik beseitigt werden. Man beachte, dass QU+QB=1.

### Bezugszeichenliste

- 1a, 1b: Stableiter
- 2a, 2b: (linkes) Bein
- 3a, 3b: (rechtes) Bein
- 4a, 4b: Mittelteil
- 5a, 5b: Endbereich
- 6a, 6b: stirnseitige Endfläche
- 7: Bearbeitungs-Laserstrahl
- 8: Schweißperle
- 9: Anbindungsfläche
- 11a, 11b: lange Seite
- 12: Kreisbahn
- 40: Anlage
- 41: Bearbeitungs-Laser
- 42: halbdurchlässiger Spiegel
- 43: Scanner-Spiegel
- 43a: programmierbare Fokussieroptik
- 44: Glühemission
- 45: Spiegel
- 45a: halbdurchlässiger Spiegel
- 46: Sensoreinrichtung
- 47: Kamera
- 48: optischer Filter
- 49: Bandpassfilter
- 50: Halteeinrichtung
- 51: Statorträger
- 52: elektronische Auswerteeinrichtung
- 53: Laserbearbeitungskopf
- 54: Bild
- 55: Teilbereich des Bildes
- 56: Teil der Schweißperle
- 57: Kollimationslinse
- 70: Beobachtungs-Lichtquelle
- 71: Beobachtungs-Lichtstrahl
- 73: Bandpassfilter
- 74: Polarisationsfilter
- 90: Photodiode
- 91: Photodiode
- 93: Bandpassfilter
- 94: Bandpassfilter
- EZ: Erstarrungszeit
- G: Grauwert
- G': erste zeitliche Ableitung des Grauwerts
- G'_{M}: mittlerer Gradient des Grauwerts
- G": zweite zeitliche Ableitung des Grauwerts
- G1, G2: Grenze (für Messvariable)
- GW: Grenzwert (für unbedeckten Anteil)
- H: Höhe der Schweißperle
- MD: Mindestdauer
- Q: Querschnitt beider Stableiter
- Qa: Querschnitt von Stableiter 1a
- Qb: Querschnitt von Stableiter 1b
- QaB: bedeckter Anteil des Querschnitts von Stableiter 1a
- QbB: bedeckter Anteil des Querschnitts von Stableiter 1b
- QaU: unbedeckter Anteil des Querschnitts von Stableiter 1a
- QbU: unbedeckter Anteil des Querschnitts von Stableiter 1b
- QU: unbedeckter Anteil des Querschnitts beider Stableiter
- SP1-SP7: Schnittpunkte
- SW1-SW7: Schwellwerte
- t: Zeit
- t₁, t₂: Zeitpunkte
- T: Temperatur
- T': erste zeitliche Ableitung der Temperatur
- T '_{M}: mittlerer Gradient der Temperatur
- T ": zweite zeitliche Ableitung der Temperatur
- T_{S}: Schmelztemperatur
- T_{V}: Verdampfungstemperatur
- ZV: zeitlicher Versatz

## Patentansprüche

1. Verfahren zur Überwachung einer Anbindungsfläche (9) beim Laserschweißen von kupferhaltigen, gebogenen Stableitern (1a, 1b), insbesondere von Hairpins für einen Elektromotor,
wobei zwei Stableiter (1a, 1b) teilweise überlappend angeordnet werden und mittels eines Bearbeitungs-Laserstrahls (7) miteinander verschweißt werden,
wobei sich eine Schweißperle (8) ausbildet, durch die die Stableiter (1a, 1b) miteinander verbunden werden,
wobei nach dem Ende der Einwirkung des Bearbeitungs-Laserstrahls (7) während des Abkühlens der Schweißperle (8) wenigstens eine mit der Temperatur der Schweißperle (8) veränderliche Messvariable zumindest an einem Teil (56) der Schweißperle (8) als Funktion der Zeit (t) gemessen wird,
**dadurch gekennzeichnet,**
**dass** aus der wenigstens einen gemessenen Messvariablen ein von der Wärmekapazität der Schweißperle (8) abhängiger Parameter bestimmt wird,
wobei über eine Kalibrierung für eine gegebene Schweißsituation aus dem Parameter auf die Anbindungsfläche geschlossen wird, und daher aus dem Parameter die Anbindungsfläche (9) qualitativ oder quantitativ bestimmt wird.

2. Verfahren nach Anspruch 1, **dadurch gekennzeichnet,**
**dass** die zwei Stableiter (1a, 1b) mit Endbereichen (5a, 5b) parallel zueinander und nebeneinander liegend angeordnet werden,
insbesondere wobei die Endbereiche (5a, 5b) der Stableiter (1a, 1b) flächig aneinander gepresst werden,
**dass** stirnseitige Endflächen (6a, 6b) der Stableiter (1a, 1b) näherungsweise auf gleicher Höhe bezüglich der Richtung einer Längserstreckung der Endbereiche (5a, 5b) der Stableiter (1a, 1b) liegen,
und **dass** der Bearbeitungs-Laserstrahl (7) so auf die zwei Stableiter (1a, 1b) gerichtet wird, dass sich die Schweißperle (8) an den stirnseitigen Endflächen (6a, 6b) der Stableiter (1a, 1b) ausbildet,
insbesondere wobei die Endbereiche (5a, 5b) der Stableiter (1a, 1b) näherungsweise vertikal nach oben gerichtet sind und der Bearbeitungs-Laserstrahl (7) näherungsweise senkrecht auf die stirnseitigen Endflächen (6a, 6b) fällt.

3. Verfahren nach Anspruch 1 oder 2, **dadurch gekennzeichnet, dass** zur Messung der Messvariable zumindest ein Teil (56) der Schweißperle (8) im sichtbaren Spektralbereich und/oder im infraroten Spektralbereich beobachtet wird.

4. Verfahren nach einem der Ansprüche 1 bis 3, **dadurch gekennzeichnet, dass** die wenigstens eine Messvariable eine Intensität einer Glühemission (44) zumindest des Teils (56) der Schweißperle (8) umfasst.

5. Verfahren nach einem der Ansprüche 1 bis 4, **dadurch gekennzeichnet, dass** während des Abkühlens der Schweißperle (8) die Schweißperle (8) mit einem Beobachtungs-Lichtstrahl (71), insbesondere Beobachtungs-Laserstrahl, beleuchtet wird,
und dass die wenigstens eine Messvariable eine Intensität des an der Oberfläche der Schweißperle (8) reflektierten Beobachtungs-Lichtstrahls (71) umfasst.

6. Verfahren nach einem der Ansprüche 4 bis 5, **dadurch gekennzeichnet, dass** die Schweißperle (8) mit einer Kamera (47) beobachtet wird, und die Intensität der Glühemission (44) und/oder des reflektierten Beobachtungs-Lichtstrahls (71) an einem Teil (56) der Schweißperle (8) mit der Kamera (47) bestimmt wird, indem ein mittlerer Grauwert (G) der Kamera (47) in einem Teilbereich (55) des von der Kamera (47) aufgenommenen Bildes (54) bestimmt wird.

7. Verfahren nach einem der Ansprüche 1 bis 3, **dadurch gekennzeichnet, dass** die Messvariable eine Temperatur (T) zumindest an einem Teil (56) der Schweißperle (8) umfasst.

8. Verfahren nach einem der vorhergehenden Ansprüche, **dadurch gekennzeichnet, dass** der Parameter eine Zeitdauer ist, die zwischen einem ersten definierten Zustand und einem zweiten definierten Zustand während des Abkühlens der Schweißperle (8) vergeht.

9. Verfahren nach Anspruch 8, **dadurch gekennzeichnet, dass** der erste definierte Zustand das Ende der Einwirkung des Bearbeitungs-Laserstrahls (7) ist.

10. Verfahren nach Anspruch 8 oder 9, **dadurch gekennzeichnet, dass** der zweite definierte Zustand die vollständige Erstarrung der Schweißperle (8) ist.

11. Verfahren nach einem der vorhergehenden Ansprüche, **dadurch gekennzeichnet, dass** der Parameter ein zeitlicher Gradient (G'_{M}, T '_{M}) der Messvariable ist, der zu einem vorgegebenen Zeitpunkt oder gemittelt über einen vorgegebenen Zeitraum bestimmt wird.

12. Verfahren nach einem der vorhergehenden Ansprüche, **dadurch gekennzeichnet,**
**dass** der Parameter oder eine aus dem Parameter quantitativ bestimmte Anbindungsfläche (9) mit einem Entscheidungswert verglichen wird, dass wenn der Entscheidungswert nicht erreicht wird, die Anbindungsfläche (9) der Verschweißung als zu klein erkannt wird, insbesondere wobei in diesem Fall betroffene Stableiter (1a, 1b) aussortiert werden oder ein Nachschweißen vorgenommen wird,
und **dass** wenn der Entscheidungswert erreicht wird, die Anbindungsfläche (9) als ausreichend groß erkannt wird und betroffene Stableiter (1a, 1b) für die weitere Verwendung zugelassen werden.

13. Verfahren nach Anspruch 12, **dadurch gekennzeichnet, dass** mittels einer Kamera (47) ein nicht von der Schweißperle (8) bedeckter Anteil (QU) eines Querschnitts (Q) der Stableiter (1a, 1b) bestimmt wird, und dass falls der nicht bedeckte Anteil (QU) des Querschnitts (Q) einen Grenzwert (GW) überschreitet, die Anbindungsfläche (9) der Verschweißung der Stableiter (1a, 1b) als zu klein erkannt wird, auch wenn der Entscheidungswert erreicht wird.

14. Verfahren nach einem der vorhergehenden Ansprüche, **dadurch gekennzeichnet, dass** eine Vielzahl von Paaren von Stableitern (1a, 1b) nacheinander verschweißt werden, wobei ein oder mehrere Schweißparameter beim Verschweißen der Paare von Stableitern (1a, 1b) in einem Regelkreis optimiert und/oder nachgeregelt werden, so dass der Parameter oder eine aus dem Parameter quantitativ bestimmte Anbindungsfläche (9) für die Paare von verschweißten Stableitern (1a, 1b) auf einen vorgegebenen Zielwert eingestellt werden.

15. Anlage (40) zum Laserschweißen von kupferhaltigen, gebogenen Stableitern (1a, 1b), insbesondere von Hairpins für einen Elektromotor, die zur Ausführung des Verfahrens nach einem der Ansprüche 1 bis 14 eingerichtet ist und die mit einer Halteeinrichtung (50), mit der zwei Stableiter (1a, 1b) überlappend angeordnet werden können, insbesondere wobei die Halteeinrichtung (50) einen Statorträger (51) mit einer Vielzahl von zu verschweißenden Stableitern (1a, 1b) umfasst, und mit einem Laserbearbeitungskopf (53) ausgestattet ist, der einen Bearbeitungs-Laserstrahl (7) bereitstellt, mit dem die zwei Stableiter (1a, 1b) miteinander verschweißt werden, so dass sich eine Schweißperle (8) ausbildet, durch die die Stableiter (1a, 1b) miteinander verbunden werden, wobei die Anlage (40) weiterhin eine Sensoreinrichtung (46) umfasst, mit der nach dem Ende der Einwirkung des Bearbeitungs-Laserstrahls (7) während des Abkühlens der Schweißperle (8) wenigstens eine mit der Temperatur der Schweißperle (8) veränderliche Messvariable zumindest an einem Teil (56) der Schweißperle (8) messbar ist,
**dadurch gekennzeichnet,**
**dass** die Anlage (40) weiterhin eine elektronische Auswerteeinrichtung (52) aufweist, die dazu eingerichtet ist, insbesondere dazu programmiert ist, aus der wenigstens einen gemessenen Messvariablen einen von der Wärmekapazität der Schweißperle (8) abhängigen Parameter zu bestimmen,
und **dass** die elektronische Auswerteeinrichtung (52) weiterhin dazu ausgebildet ist, insbesondere dazu programmiert ist, aus dem Parameter eine
durch die Schweißperle (8) eingerichtete Anbindungsfläche (9) qualitativ oder quantitativ zu bestimmen, wobei über eine Kalibrierung für eine gegebene Schweißsituation aus dem Parameter auf die Anbindungsfläche geschlossen wird.

## Claims

1. A method for monitoring a connection area (9) during laser welding of copper-containing bent conductor bars (1a, 1b), in particular hairpins for an electric motor, wherein two conductor bars (1a, 1b) are arranged in a partially overlapping manner and welded together by means of a machining laser beam (7),
wherein a weld bead (8) is formed, via which the conductor bars (1a, 1b) are connected to each other,
wherein, after the action of the machining laser beam (7) has been terminated, during the cooling of the weld bead (8), at least one measured variable which varies with the temperature of the weld bead (8) is measured at least at a part (56) of the weld bead (8) as a function of time (t),
**characterised in that**
a parameter dependent on the heat capacity of the weld bead (8) is determined from at least one measured variable,
wherein, via a calibration for a given welding situation, the connection area is inferred from the parameter, and therefore the connection area (9) is qualitatively or quantitatively determined from the parameter.

2. The method according to Claim 1, **characterised**
**in that** the two conductor bars (1a, 1b) with end regions (5a, 5b) are arranged parallel to each other and next to each other,
in particular wherein the end regions (5a, 5b) of the conductor bar (1a, 1b) are pressed together over a flat surface,
**in that** the end faces (6a, 6b) of the conductor bar (1a, 1b) are approximately at the same height with respect to the direction of a longitudinal extension of the end regions (5a, 5b) of the conductor bar (1a, 1b),
and **in that** the machining laser beam (7) is directed at the two conductor bars (1a, 1b) in such a way that the weld bead (8) forms on the end faces (6a, 6b) of the conductor bars (1a, 1b),
in particular wherein the end regions (5a, 5b) of the conductor bar (1a, 1b) are directed approximately vertically upwards and the machining laser beam (7) falls approximately perpendicularly onto the end faces (6a, 6b).

3. The method according to Claim 1 or 2, **characterised in that** at least a part (56) of the weld bead (8) is observed in the visible spectral range and/or in the infrared spectral range for measuring the measured variable.

4. The method according to any one of Claims 1 to 3, **characterised in that** the at least one measured variable comprises an intensity of a thermionic emission (44) of at least part (56) of the weld bead (8).

5. The method according to any one of Claims 1 to 4, **characterised in that**, during the cooling of the weld bead (8), the weld bead (8) is illuminated with an observation light beam (71), in particular an observation laser beam,
and **in that** at least one measured variable comprises an intensity of the observation light beam (71) reflected at the surface of the weld bead (8).

6. The method according to one of Claims 4 to 5, **characterised in that** the weld bead (8) is observed with a camera (47), and the intensity of the thermionic emission (44) and/or the reflected observation light beam (71) at a part (56) of the weld bead (8) is determined with the camera (47) by determining a mean greyscale value (G) of the camera (47) in a subregion (55) of the image (54) taken by the camera (47).

7. The method according to any one of Claims 1 to 3, **characterised in that** the measured variable comprises a temperature (T) at least at a part (56) of the weld bead (8).

8. The method according to any one of the preceding claims, **characterised in that** the parameter is a time period that elapses between a first defined state and a second defined state during the cooling of the weld bead (8).

9. The method according to Claim 8, **characterised in that** the first defined state is when the action of the machining laser beam (7) is terminated.

10. The method according to Claim 8 or 9, **characterised in that** the second defined state is the complete solidification of the weld bead (8).

11. The method according to any one of the preceding claims, **characterised in that** the parameter is a temporal gradient (G'_{M}, T'_{M}) of the measured variable, which is determined at a predetermined time or averaged over a predetermined time period.

12. The method according to any one of the preceding claims, **characterised in that** the parameter or a connection area (9) quantitatively determined from the parameter is compared with a decision value,
**in that** if the decision value is not reached, the connection area (9) of the weld is recognised as being too small, in particular in which case affected conductor bars (1a, 1b) are sorted out or re-welding is carried out,
and **in that** when the decision value is reached, the connection area (9) is deemed to be sufficiently large and the affected conductor bars (1a, 1b) are approved for further use.

13. The method according to Claim 12, **characterised in that** a portion (QU) of a cross-section (Q) of the conductor bar (1a, 1b) not covered by the weld bead (8) is determined by means of a camera (47), and **in that** if the uncovered portion (QU) of the cross-section (Q) exceeds a limit value (GW), the connection area (9) of the welding of the conductor bar (1a, 1b) is recognised as being too small, even if the decision value is reached.

14. The method according to any one of the preceding claims, **characterised in that** a plurality of pairs of conductor bars (1a, 1b) are welded one after the other, wherein one or more welding parameters are optimised and/or adjusted in a control loop during the welding of the pairs of conductor bars (1a, 1b), so that the parameter or a connection area (9) quantitatively determined from the parameter for the pairs of welded conductor bars (1a, 1b) is set to a predetermined target value.

15. A system (40) for laser welding copper-containing, bent conductor bars (1a, 1b), in particular hairpins for an electric motor, configured for carrying out the method according to any one of claims 1 to 14, and comprising a holding device (50) with which two conductor bars (1a, 1b) can be arranged in an overlapping manner, in particular wherein the holding device (50) comprises a stator carrier (51) with a plurality of conductor bars (1a, 1b) to be welded, and is equipped with a laser machining head (53) that provides a machining laser beam (7) with which the two conductor bars (1a, 1b) are welded together, so that a weld bead (8) is formed by means of which the conductor bars (1a, 1b) are connected to each other, wherein the system (40) further comprises a sensor device (46) with which, after the action of the machining laser beam (7) has been terminated, during the cooling of the weld bead (8), at least one measured variable that varies with the temperature of the weld bead (8), can be measured at least at a part (56) of the weld bead (8),
**characterised**
**in that** the system (40) further has an electronic evaluation device (52) which is designed, in particular programmed, to determine a parameter dependent on the heat capacity of the weld bead (8) from the at least one measured variable,
and **in that** the electronic evaluation device (52) is further designed, in particular programmed, to qualitatively or quantitatively determine from the parameter a connection area (9) provided by the weld bead (8), wherein the connection area is inferred from the parameter via a calibration for a given welding situation.

## Revendications

1. Procédé de surveillance d'une surface de jonction (9) lors du soudage laser de barres conductrices (1a, 1b) cintrées contenant du cuivre, en particulier de conducteurs en épingles à cheveux pour un moteur électrique,
dans lequel deux barres conductrices (1a, 1b) sont disposées en se chevauchant partiellement et soudées l'une à l'autre à l'aide d'un faisceau laser d'usinage (7), dans lequel une perle de soudure (8) se forme, au moyen de laquelle les barres conductrices (1a, 1b) sont liées l'une à l'autre,
dans lequel, après la fin de l'exposition au faisceau laser d'usinage (7) lors du refroidissement de la perle de soudure (8), au moins une variable mesurée variant en fonction de la température de la perle de soudure (8) est mesurée au moins sur une partie (56) de la perle de soudure (8) en fonction du temps (t),
**caractérisé en ce**
**qu'**un paramètre dépendant de la capacité thermique de la perle de soudure (8) est déterminé à partir de l'au moins une variable mesurée,
dans lequel, à l'aide d'un étalonnage pour une situation de soudage donnée, la surface de jonction est déduite du paramètre, et par conséquent la surface de jonction (9) est déterminée qualitativement ou quantitativement à partir dudit paramètre.

2. Procédé selon la revendication 1, **caractérisé en ce**
**que** les deux barres conductrices (1a, 1b) comportant des zones d'extrémité (5a, 5b) sont disposées parallèlement l'une à l'autre et adjacentes,
en particulier dans lequel les zones d'extrémité (5a, 5b) des barres conductrices (1a, 1b) sont pressées à plat l'une contre l'autre,
en ce que les surfaces d'extrémité (6a, 6b) des barres conductrices (1a, 1b) sont situées approximativement à la même hauteur par rapport à la direction d'une extension longitudinale des zones d'extrémité (5a, 5b) des barres conductrices (1a, 1b),
et en ce que le faisceau laser d'usinage (7) est dirigé vers les deux barres conductrices (1a, 1b) de telle sorte que la perle de soudure (8) se forme sur les surfaces d'extrémité (6a, 6b) côté frontal des barres conductrices (1a, 1b),
en particulier dans lequel les zones d'extrémité (5a, 5b) des barres conductrices (1a, 1b) sont dirigées approximativement verticalement vers le haut et le faisceau laser d'usinage (7) tombe approximativement perpendiculairement sur les surfaces d'extrémité (6a, 6b) côté frontal.

3. Procédé selon la revendication 1 ou 2, **caractérisé en ce que** pour mesurer la variable mesurée, au moins une partie (56) de la perle de soudure (8) est observée dans le domaine visible du spectre et/ou dans le domaine infrarouge du spectre.

4. Procédé selon l'une quelconque des revendications 1 à 3, **caractérisé en ce que** l'au moins une variable mesurée comprend une intensité d'une émission thermoélectronique (44) d'au moins la partie (56) de la perle de soudure (8).

5. Procédé selon l'une quelconque des revendications 1 à 4, **caractérisé en ce que** lors du refroidissement de la perle de soudure (8), la perle de soudure (8) est éclairée par un faisceau lumineux d'observation (71), en particulier un faisceau laser d'observation,
et **en ce que** l'au moins une variable mesurée comprend une intensité du faisceau lumineux d'observation (71) réfléchie à la surface de la perle de soudure (8).

6. Procédé selon l'une quelconque des revendications 4 à 5, **caractérisé en ce que** la perle de soudure (8) est observée à l'aide d'une caméra (47), et l'intensité de l'émission thermoélectronique (44) et/ou du faisceau lumineux d'observation (71) réfléchi sur la partie (56) de la perle de soudure (8) est déterminée à l'aide de la caméra (47) de telle sorte qu'une valeur de niveaux de gris (G) moyenne de la caméra (47) est déterminée dans une zone partielle (55) d'une image (54) prise par la caméra (47).

7. Procédé selon l'une quelconque des revendications 1 à 3, **caractérisé en ce que** la variable mesurée comprend une température (T) au moins sur la partie (56) de la perle de soudure (8).

8. Procédé selon l'une quelconque des revendications précédentes, **caractérisé en ce que** le paramètre est une période qui s'écoule entre un premier état défini et un second état défini lors du refroidissement de la perle de soudure (8).

9. Procédé selon la revendication 8, **caractérisé en ce que** le premier état défini est la fin de l'exposition au faisceau laser d'usinage (7).

10. Procédé selon la revendication 8 ou 9, **caractérisé en ce que** le second état défini est la solidification complète de la perle de soudure (8).

11. Procédé selon l'une quelconque des revendications précédentes, **caractérisé en ce que** le paramètre est un gradient temporel (G'_{M}, T'_{M}) de la variable mesurée, lequel est déterminé à un moment prédéterminé ou moyenné sur une période prédéterminée.

12. Procédé selon l'une quelconque des revendications précédentes, **caractérisé en ce que** le paramètre ou la surface de jonction (9) déterminée quantitativement à partir du paramètre sont comparés à une valeur d'état,
**en ce que**, lorsque la valeur d'état n'est pas atteinte, la surface de jonction (9) de la soudure est considérée comme étant trop petite, en particulier les barres conductrices (1a, 1b) concernées étant dans ce cas triées ou un nouveau soudage étant effectué,
et **en ce que** lorsque la valeur d'état est atteinte, la surface de jonction (9) est considérée comme suffisamment grande et les barres conductrices (1a, 1b) concernées sont approuvées pour une utilisation ultérieure.

13. Procédé selon la revendication 12, **caractérisé en ce que**, au moyen de la caméra (47), une fraction (QU) d'une section transversale (Q) de la barre conductrice (1a, 1b) non recouverte par la perle de soudure (8) est déterminée, et **en ce que**, lorsque la fraction (QU) non recouverte de la section transversale (Q) est supérieure à une valeur limite (GW), la surface de jonction (9) de la soudure des barres conductrices (1a, 1b) est reconnue comme trop petite, même si la valeur d'état est atteinte.

14. Procédé selon l'une quelconque des revendications précédentes, **caractérisé en ce qu'**une pluralité de paires de barres conductrices (1a, 1b) sont soudées les unes après les autres, dans lequel au moins un paramètre de soudage est optimisé et/ou réglé dans un circuit de régulation lors du soudage des paires de barres conductrices (1a, 1b) de telle sorte que le paramètre ou la surface de jonction (9) déterminé quantitativement à partir du paramètre pour les paires de barres conductrices (1a, 1b) soudées sont fixés à une valeur cible prédéterminée.

15. Installation (40) de soudage laser de barres conductrices (1a, 1b) cintrées contenant du cuivre, en particulier de conducteurs en épingle à cheveux pour un moteur électrique, conçue pour mettre en œuvre le procédé selon l'une quelconque des revendications 1 à 14, et comprenant un dispositif de maintien (50) à l'aide duquel deux barres conductrices (1a, 1b) peuvent être disposées de manière superposée, en particulier dans laquelle le dispositif de maintien (50) comprend un support de stator (51) comportant une pluralité de barres conductrices (1a, 1b) à souder et est doté d'une tête d'usinage laser (53), laquelle fournit un faisceau laser d'usinage (7) à l'aide duquel les deux barres conductrices (1a, 1b) peuvent être soudées l'une à l'autre de telle sorte qu'une perle de soudure (8) se forme, au moyen de laquelle les barres conductrices (1a, 1b) sont liées l'une à l'autre, l'installation (40) comprenant en outre un dispositif capteur (46) au moyen duquel, après la fin de l'exposition au faisceau laser d'usinage (7), lors du refroidissement de la perle de soudure (8), au moins une variable mesurée variant en fonction de la température de la perle de soudure (8) est mesurable au moins sur une partie (56) de la perle de soudure (8),
**caractérisée en ce**
**que** l'installation (40) comprend en outre un dispositif d'évaluation (52) électronique, lequel est conçu, en particulier programmé, pour déterminer, à partir de l'au moins une variable mesurée, un paramètre dépendant de la capacité thermique de la perle de soudure (8),
et en ce que le dispositif d'évaluation (52) électronique est en outre conçu, en particulier programmé, pour déterminer qualitativement ou quantitativement, à partir du paramètre, une surface de jonction (9) conçue par la perle de soudure (8), dans laquelle la surface de jonction est déduite du paramètre à l'aide d'un étalonnage pour une situation de soudage donnée.
